(19) Europäisches Patentamt / European Patent Office / Office européen des brevets

(11) **EP 2 398 909 B1**

(12) **EUROPÄISCHE PATENTSCHRIFT**

(45) Veröffentlichungstag und Bekanntmachung des Hinweises auf die Patenterteilung:
**22.07.2015 Patentblatt 2015/30**

(21) Anmeldenummer: **10704923.1**

(22) Anmeldetag: **12.02.2010**

(51) Int Cl.:
***C12Q 1/32*** *(2006.01)* ***C12Q 1/54*** *(2006.01)*
***C12N 9/04*** *(2006.01)*

(86) Internationale Anmeldenummer:
**PCT/EP2010/051801**

(87) Internationale Veröffentlichungsnummer:
**WO 2010/094632 (26.08.2010 Gazette 2010/34)**

(54) **SCHNELLE REAKTIONSKINETIK VON ENZYMEN MIT NIEDRIGER AKTIVITÄT IN TROCKENEN CHEMIESCHICHTEN**

FAST REACTION KINETICS OF ENZYMES HAVING LOW ACTIVITY IN DRY CHEMISTRY LAYERS

CINÉTIQUE RÉACTIONNELLE RAPIDE D'ENZYMES PRÉSENTANT UNE ACTIVITÉ INFÉRIEURE DANS DES COUCHES CHIMIQUES SÈCHES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **19.02.2009 PCT/EP2009/001206**

(43) Veröffentlichungstag der Anmeldung:
**28.12.2011 Patentblatt 2011/52**

(73) Patentinhaber:
- **F. Hoffmann-La Roche AG**
  **4070 Basel (CH)**
  Benannte Vertragsstaaten:
  **AT BE BG CH CY CZ DK EE ES FI FR GB GR HR HU IE IS IT LI LT LU LV MC MK MT NL NO PL PT RO SE SI SK SM TR**
- **Roche Diagnostics GmbH**
  **68305 Mannheim (DE)**
  Benannte Vertragsstaaten:
  **DE**

(72) Erfinder:
- **HORN, Carina**
  **68647 Biblis (DE)**
- **GÄSSLER-DIETSCHE, Claudia**
  **69198 Schriesheim (DE)**
- **HEINDL, Dieter**
  **82396 Pähl (DE)**
- **HÖNES, Joachim**
  **64673 Zwingenberg (DE)**
- **MEIER, Thomas**
  **81373 München (DE)**
- **SCHMUCK, Rainer**
  **83671 Benediktbeuern (DE)**

(74) Vertreter: **Weickmann & Weickmann**
**Postfach 860 820**
**81635 München (DE)**

(56) Entgegenhaltungen:
**WO-A1-2007/012494 WO-A1-2009/103540**

- **BAIK SANG-HO ET AL: "Cooperative effect of two surface amino acid mutations (Q252L and E170K) in glucose dehydrogenase from Bacillus megaterium IWG3 on stabilization of its oligomeric state" Juni 2005 (2005-06), APPLIED AND ENVIRONMENTAL MICROBIOLOGY, VOL. 71, NR. 6, PAGE(S) 3285-3293 , XP002580338 ISSN: 0099-2240 in der Anmeldung erwähnt Zusammenfassung**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 398 909 B1

Printed by Jouve, 75001 PARIS (FR)

**Beschreibung**

Die vorliegende Erfindung betrifft ein Verfahren zur Bestimmung eines Analyten sowie ein hierzu geeignetes diagnostisches Element.

Diagnostische Elemente sind wichtige Bestandteile klinisch relevanter Analyseverfahren. Hierbei steht die Messung von Analyten, z.B. Metaboliten oder Substraten, im Vordergrund, welche beispielsweise mit Hilfe eines für den Analyten spezifischen Enzyms direkt oder indirekt bestimmt werden. Die Analyten werden hierbei mit Hilfe eines Enzym-Coenzym-Komplexes umgesetzt und anschließend quantifiziert. Dabei wird der zu bestimmende Analyt mit einem geeigneten Enzym, einem Coenzym und gegenenfalls einem Mediator in Kontakt gebracht, wobei das Coenzym durch die enzymatische Reaktion physikochemisch verändert, z.B. oxidiert bzw. reduziert, wird. Sofern zusätzlich ein Mediator zum Einsatz gelangt, überträgt dieser die bei Umsetzung des Analyten freigesetzten Elektronen vom reduzierten Coenzym üblicherweise auf einen optischen Indikator oder die leitfähigen Bestandteile einer Elektrode, so dass der Vorgang beispielsweise photometrisch oder elektrochemisch erfasst werden kann. Eine Kalibrierung liefert einen direkten Zusammenhang des Messwerts mit der Konzentration des zu bestimmenden Analyten.

Aus dem Stand der Technik bekannte diagnostische Elemente zeichnen sich durch eine zeitlich begrenzte Haltbarkeit sowie durch spezielle Anforderungen an die Umgebung, wie Kühlung oder trockene Lagerung, zur Erzielung dieser Haltbarkeit aus. Bei bestimmten Anwendungsformen, z.B. bei Tests, die vom Endverbraucher selbst durchgeführt werden, wie etwa beim Blutglucose-Selbstmonitoring, können daher durch eine falsche, unbemerkte Fehllagerung des Messsystems Fehlergebnisse vorkommen, welche vom Verbraucher kaum zu erkennen sind und ggf. zu einer Fehlbehandlung der jeweiligen Erkrankung führen können. Die Fehlergebnisse beruhen in erster Linie auf der Tatsache, dass die in derartigen diagnostischen Elementen eingesetzten Enzyme, Coenzyme und Mediatoren im Allgemeinen empfindlich auf Feuchtigkeit und Wärme reagieren und mit der Zeit inaktiviert werden.

Eine bekannte Maßnahme, die zur Erhöhung der Stabilität von diagnostischen Elementen eingesetzt wird, ist die Verwendung stabiler Enzyme, z.B. die Verwendung von Enzymen aus thermophilen Organismen. Weiterhin besteht die Möglichkeit, Enzyme durch chemische Modifizierung, insbesondere durch Quervernetzung, zu stabilisieren. Darüber hinaus können auch Enzymstabilisatoren, wie z.B. Trehalose, Polyvinylpyrrolidon und Serumalbumin, zugesetzt werden oder die Enzyme z.B. durch Photopolymerisation in Polymernetzwerke eingeschlossen werden.

Eine weitere Möglichkeit der Stabilisierung von Enzymen bieten ortsspezifisch oder nicht-ortsspezifisch eingeführte Mutationen. Als besonders geeignet hat sich in diesem Zusammenhang die Verwendung rekombinanter Techniken erwiesen, welche durch gezielte Veränderung der für ein Enzym kodierenden DNA eine gezielte Beeinflussung der Eigenschaften des entsprechenden Enzyms ermöglichen.

Baik et al. (Appl. Environ. Microbiol. (2005), 71, 3285) beschreiben die Isolierung und Charakterisierung dreier Mutanten von Glucose-Dehydrogenase aus Bacillus megaterium, welche die Aminosäuresubstitutionen E170K, Q252L bzw. E170K/Q252L enthalten. Während die Mutanten E170K und Q252L bei niedrigen Salzkonzentrationen und hohen pH-Werten lediglich eine geringe Stabilität besitzen, zeigte sich für die Doppelmutante aufgrund einer verstärkten Wechselwirkung an der Dimer-Dimer-Grenzfläche unter den Testbedingungen eine signifikant erhöhte Stabilität.

Vázquez-Figueroa et al. (ChemBioChem (2007), 8, 2295) offenbaren die Entwicklung einer thermostabilen Glucose-Dehydrogenase, welche das Einbringen von Aminosäuresubstitutionen an den Positionen 155, 170 und 252 von Glucose-Dehydrogenase aus Bacillus subtilis, Bacillus thuringiensis und Bacillus licheniformis umfasst. In diesem Zusammenhang ist ausgeführt, dass die Mutationen E170K und Q252L, sowohl einzeln als auch in Kombination, zu einer Stabilisierung von Glucose-Dehydrogenase aus Bacillus subtilis führen.

Bei der Verwendung stabilisierter, gegenüber der Wildtyp-Variante genetisch veränderter Enzyme ergibt sich indessen das Problem, dass diese im Regelfall eine deutlich geringere Aktivität als das entsprechende Wildtyp-Enzym aufweisen und damit einen geringeren Substratumsatz pro Zeiteinheit bewirken. Unter Berücksichtigung der Tatsache, dass in der klinischen und diagnostischen Chemie, beispielsweise beim Nachweis von Blutglucose, bevorzugt Enzyme mit einer hohen spezifischen Aktivität zur Anwendung gelangen, stellt der Einsatz stabilisierter Enzyme eine häufig inakzeptable Alternative zur Verwendung nativer Enzyme dar.

Weiterhin besteht die Schwierigkeit, dass hohe Enzymaktivitäten, welche mit einem hohen Substratumsatz pro Zeiteinheit korrelieren, in der Regel nur mit dem jeweils zugehörigen, nativen Coenzym erreicht werden. Verwendet man an Stelle des nativen Coenzyms ein artifizielles Coenzym, so verringert sich die Enzymaktivität im Allgemeinen drastisch und die Umsatzrate mit dem Substrat sinkt entsprechend.

Die der vorliegenden Erfindung zugrunde liegende Aufgabe bestand somit darin, ein stabiles diagnostisches Element, insbesondere zur Bestimmung von Glucose, bereitzustellen, bei welchem die Nachteile des Standes der Technik zumindest teilweise beseitigt sind. Insbesondere sollte das diagnostische Element bei gleichzeitig hoher Stabilität sowohl des Enzyms als auch des Coenzyms eine hohe Umsatzgeschwindigkeit des Substrats gewährleisten.

Diese Aufgabe wird erfindungsgemäß gelöst durch ein Verfahren zur Bestimmung eines Analyten, umfassend die Schritte:

(a) Inkontaktbringen einer den Analyten enthaltenden Probe mit einem diagnostischen Element, umfassend eine trockene Reagenzienschicht, welche

(i) eine für den Analyten spezifische, mutierte Dehydrogenase und
(ii) ein artifizielles Coenzym enthält, und

(b) Bestimmen des Vorhandenseins oder/und der Menge des Analyten, wobei die mutierte Glucose-Dehydrogenase eine gegenüber dem korrespondierenden Wildtyp-Enzym erhöhte thermische oder hydrolytische Stabilität aufweist und eine Mutation an Position 170 oder/und Position 252 der Aminosäuresequenz der korrespondierenden Wildtyp-Glucose-Dehydrogenase umfasst, und wobei das artifizielle Coenzym eine im Vergleich zum nativen Coenzym erhöhte hydrolytische Stabilität aufweist und eine Verbindung der Formel (II) wie im angehängten Anspruch 1 und auf Seite 9 definiert, ist.

Überraschenderweise wurde im Rahmen der vorliegenden Erfindung festgestellt, dass mutierte Dehydrogenasen, welche in Gegenwart eines artifiziellen Coenzyms im Küvettentest äußerst geringe Aktivitäten aufweisen, in diagnostischen Elementen mit trockenen Reagenzschichten, wie beispielsweise in Teststreifen, eine schnellere Kinetik zeigen und mindestens genauso viel Umsatz liefern wie in Gegenwart des nativen Coenzyms (Wildtyp-Coenzym). Die Ursache hierfür liegt vermutlich darin begründet, dass bei hohen Konzentrationen der Einsatzstoffe andere Faktoren als die Aktivität des Enzyms die Umsatzgeschwindigkeit entscheidend beeinflussen, wobei in diesem Zusammenhang insbesondere die Lage der Komplexbildung zwischen Enzym, Coenzym, reduziertem Coenzym, Analyt und oxidiertem Analyt ausschlaggebend zu sein scheint. Insofern sieht das erfindungsgemäße Verfahren in einer bevorzugten Ausführungsform vor, dass die Umsatzgeschwindigkeit des Analyten in dem hierin beschriebenen diagnostischen Element gegenüber der Umsatzgeschwindigkeit des Analyten in einem korrespondierenden diagnostischen Element, welches anstelle des artifiziellen Coenzyms das korrespondierende Wildtyp-Coenzym umfasst, gleich oder erhöht ist. Bevorzugt ist die Umsatzgeschwindigkeit des Analyten in einem erfindungsgemäß verwendeten diagnostischen Element im Vergleich zur Umsatzgeschwindigkeit des Analyten in einem das Wildtyp-Coenzym umfassenden diagnostischen Element um mindestens 20%, stärker bevorzugt um mindestens 50%, und am stärksten bevorzugt um mindestens 100%, beispielsweise um 100% bis 200%, erhöht.

Die Begriffe "mutierte Dehydrogenase" oder "Dehydrogenase-Mutante", wie sie in vorliegender Anmeldung verwendet werden, bezeichnen eine genetisch veränderte Variante einer nativen Dehydrogenase (Wildtyp-Dehydrogenase), welche bei gleicher Anzahl an Aminosäuren eine gegenüber der Wildtyp-Dehydrogenase veränderte Aminosäuresequenz besitzt, d.h. sich in mindestens einer Aminosäure von der Wildtyp-Dehydrogenase unterscheidet. Die mutierte Glucose-Dehydrogenase umfasst eine Mutation an Position 170 oder/und Position 252 der Aminosäuresequenz der korrespondierenden Wildtyp-Glucose-Dehydrogenase.

Die mutierte Dehydrogenase kann durch Mutation einer aus einer beliebigen biologischen Quelle stammenden Wildtyp-Dehydrogenase erhalten werden, wobei der Begriff "biologische Quelle" im Sinne dieser Erfindung sowohl Prokaryonten, wie beispielsweise Bakterien, als auch Eukaryonten, wie beispielsweise Säuger und andere Tiere, umfasst. Die Einführung der Mutation(en) kann dabei ortsspezifisch oder nicht-ortsspezifisch, bevorzugt ortsspezifisch unter Verwendung von im Fachbereich bekannten rekombinanten Methoden, erfolgen, wobei entsprechend den jeweiligen Anforderungen und Bedingungen mindestens ein Aminosäureaustausch innerhalb der Aminosäuresequenz der nativen Dehydrogenase resultiert.

Eine auf diese Weise erhaltene, in dem erfindungsgemäßen Verfahren eingesetzte Dehydrogenase-Mutante weist eine gegenüber der korrespondierenden Wildtyp-Dehydrogenase erhöhte thermische oder/und hydrolytische Stabilität auf. Beispiele für derartige Mutanten sind u.a. in Baik (Appl. Environ. Microbiol. (2005), 71, 3285), Vásquez-Figueroa (ChemBioChem (2007), 8, 2295) sowie in WO 2005/045016 A2 beschrieben, auf deren Offenbarung hiermit ausdrücklich Bezug genommen wird.

Besonders bevorzugt besitzt eine mutierte Dehydrogenase im Sinne der vorliegenden Erfindung eine gegenüber der korrespondierenden Wildtyp-Dehydrogenase verringerte spezifische Enzymaktivität. Der Begriff "spezifische Enzymaktivität" (angegeben in U/mg Enzym), wie er in vorliegender Anmeldung verwendet wird, bezeichnet dabei die Menge an Substrat, welche unter vorbestimmten Bedingungen pro Minute und pro Milligramm an Enzym umgesetzt wird. Der Begriff "Lyophilisat-Aktivität" bezeichnet demgegenüber die Menge an Substrat, welche unter vorbestimmten Bedingungen pro Minute und pro Milligramm an Lyophilisat, umfassend das Enzym in Kombination mit Hilfsstoffen, umgesetzt wird.

Die in dem offenbarten Verfahren eingesetzte mutierte Dehydrogenase ist vorzugsweise eine Nikotinamid-Adenin-Dinukleotid (NAD/NADH)- oder Nikotinamid-Adenin-Dinukleotidphosphat (NADP/NADPH)-abhängige mutierte Dehydrogenase, welche bevorzugt ausgewählt ist aus einer mutierten Alkohol-Dehydrogenase (EC 1.1.1.1; EC 1.1.1.2), einer mutierten L-Aminosäure-Dehydrogenase (EC 1.4.1.5), einer mutierten Glucose-Dehydrogenase (EC 1.1.1.47), einer mutierten Glucose-6-phosphat-Dehydrogenase (EC 1.1.1.49), einer mutierten Glycerin-Dehydrogenase (EC

1.1.1.6), einer mutierten 3-Hydroxybutyrat-Dehydrogenase (EC 1.1.1.30), einer mutierten Lactat-Dehydrogenase (EC 1.1.1.27; EC 1.1.1.28), einer mutierten Malat-Dehydrogenase (EC 1.1.1.37) und einer mutierten Sorbitol-Dehydrogenase. Erfindungsgemäß handelt es sich bei der mutierten Dehydrogenase um eine mutierte Glucose-Dehydrogenase (EC 1.1.1.47).

Sofern im Rahmen des erfindungsgemäßen Verfahrens eine mutierte Glucose-Dehydrogenase eingesetzt wird, so umfasst diese eine Mutation an mindestens einer der Positionen 170 und 252 der Aminosäuresequenz der Wildtyp-Glucose-Dehydrogenase, wobei Mutanten mit Mutationen an Position 170 und Position 252 besonders bevorzugt sind. Als vorteilhaft hat es sich erwiesen, wenn die mutierte Glucose-Dehydrogenase neben diesen Mutationen keine weiteren Mutationen enthält.

Die Mutation an den Positionen 170 oder/und 252 kann grundsätzlich einen beliebigen Aminosäureaustausch umfassen, welcher zu einer Stabilisierung, z.B. einer Erhöhung der thermischen oder/und hydrolytischen Stabilität, der Wildtyp-Dehydrogenase führt. Vorzugsweise umfasst die Mutation an Position 170 einen Aminosäureaustausch von Glutaminsäure gegen Arginin oder Lysin, insbesondere einen Aminosäureaustausch von Glutaminsäure gegen Lysin, während in Bezug auf Position 252 ein Aminosäureaustausch von Lysin gegen Leucin bevorzugt ist.

Die zur Herstellung obiger Mutanten von Glucose-Dehydrogenase verwendeten Wildtyp-Glucose-Dehydrogenasen entstammen vorzugsweise einem Bakterium, wobei besonders bevorzugt eine Glucose-Dehydrogenase aus Bacillus megaterium, Bacillus subtilis oder Bacillus thuringiensis, insbesondere aus Bacillus subtilis, zur Anwendung gelangt. In der am stärksten bevorzugten Ausführungsform wird im Rahmen des erfindungsgemäßen Verfahrens eine durch Mutation von Wildtyp-Glucose-Dehydrogenase aus Bacillus subtilis erhaltene mutierte Glucose-Dehydrogenase GlucDH_E170K_K252L mit der in SEQ ID NO: 1 dargestellten Aminosäuresequenz verwendet.

Erfindungsgemäß umfassen die hierin beschriebenen diagnostischen Elemente neben einer für den Analyten spezifischen, mutierten Dehydrogenase weiterhin ein artifizielles Coenzym. Ein artifizielles Coenzym im Sinne der vorliegenden Erfindung ist ein gegenüber dem nativen Coenzym chemisch verändertes Coenzym, welches bei Atmosphärendruck eine im Vergleich zum nativen Coenzym höhere Stabilität gegenüber Feuchtigkeit, Temperaturen insbesondere im Bereich von 0°C bis 50°C, Säuren und Basen insbesondere im Bereich von pH 4 bis pH 10, oder/und Nukleophilen wie beispielsweise Alkoholen oder Aminen, aufweist und insofern unter identischen Umgebungsbedingungen über einen längeren Zeitraum als das native Coenzym seine Wirkung entfalten kann. Das artifizielle Coenzym weist eine im Vergleich zum nativen Coenzym höhere hydrolytische Stabilität auf, wobei eine vollständige

Hydrolysestabilität unter Testbedingungen besonders bevorzugt ist. Im Vergleich zum nativen Coenzym kann das artifizielle Coenzym eine verringerte Bindungskonstante für die Dehydrogenase aufweisen, beispielsweise eine um den Faktor von 2 oder mehr verringerte Bindungskonstante.

Bevorzugte Beispiele für artifizielle Coenzyme, welche im Rahmen des offenbarten Verfahrens eingesetzt werden können, sind artifizielle NAD(P)/NAD(P)H-Verbindungen, d.h. chemische Derivate von nativem Nikotinamid-Adenin-Dinukleotid (NAD/NADH) bzw. nativem Nikotinamid-Adenin-Dinukleotidphosphat (NADP/NADPH), oder die Verbindung der Formel (I)

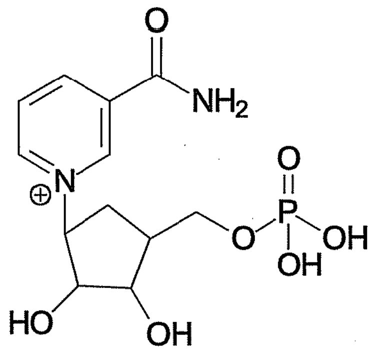

(I)

Sofern es sich bei dem artifiziellen Coenzym um eine artifizielle NAD(P)/NAD(P)H-Verbindung handelt, umfasst die artifizielle NAD(P)/NAD(P)H-Verbindung vorzugsweise einen 3-Pyridincarbonyl- oder einen 3-Pyridinthiocarbonyl-Rest, welcher ohne glykosidische Bindung über einen linearen oder cyclischen organischen Rest, insbesondere über einen cyclischen organischen Rest, mit einem phosphorhaltigen Rest, wie beispielsweise einem Phosphatrest, verknüpft ist.

Im erfindungsgemäßen Verfahren wird das artifizielle Coenzym aus einer Verbindung der allgemeinen Formel (II) verwendet

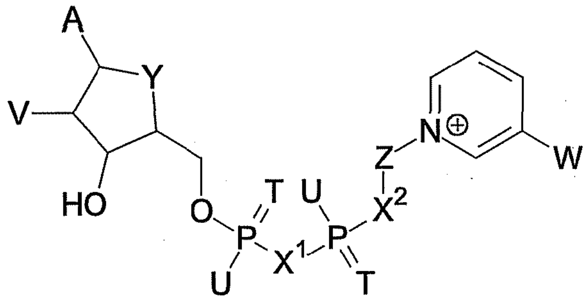

(II)

worin

| | |
|---|---|
| A = | Adenin oder ein Analogon davon, |
| T = | jeweils unabhängig O, S, |
| U = | jeweils unabhängig OH, SH, $BH_3^-$, $BCNH_2^-$, |
| V = | jeweils unabhängig OH oder eine Phosphatgruppe, oder zwei Gruppen, die eine cyclische Phosphatgruppe bilden; |
| W = | COOR, $CON(R)_2$, COR, $CSN(R)_2$ mit R = jeweils unabhängig H oder $C_1$-$C_2$-Alkyl, |
| $X^1$, $X^2$ = | jeweils unabhängig O, $CH_2$, $CHCH_3$, $C(CH_3)_2$, NH, $NCH_3$, |
| Y = | NH, S, O, $CH_2$, |
| Z = | ein linearer oder cyclischer organischer Rest ist, mit der Maßgabe, dass Z und der Pyridin-Rest nicht durch eine glycosidische Verbindung verknüpft sind, oder ein Salz oder gegebenenfalls eine reduzierte Form davon. |

In den Verbindungen der Formel (II) ist Z vorzugsweise ein linearer Rest mit 4-6 C-Atomen, vorzugsweise mit 4 C-Atomen, worin 1 oder 2 C-Atome gegebenenfalls durch ein oder mehrere Heteroatome ausgewählt aus O, S und N ersetzt sind, oder ein Rest umfassend eine cyclische Gruppe mit 5 oder 6 C-Atomen, die gegebenenfalls ein Heteroatom ausgewählt aus O, S und N sowie gegebenenfalls einen oder mehrere Substituenten enthält, und einen Rest $CR^4_2$, wobei $CR^4_2$ an die cyclische Gruppe und an $X^2$ gebunden ist, mit $R^4$ = jeweils unabhängig H, F, Cl, $CH_3$.

Besonders bevorzugt ist Z ein gesättigter oder ungesättigter carbocyclischer oder heterocyclischer Fünfring, insbesondere eine Verbindung der allgemeinen Formel (III),

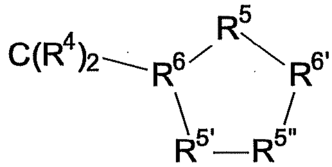

(III)

wobei zwischen $R^{5'}$ und $R^{5''}$ eine Einfach- oder Doppelbindung vorliegen kann, mit

| | |
|---|---|
| $R^4$ = | jeweils unabhängig H, F, Cl, $CH_3$, |
| $R^5$ = | $CR^4_2$, |
| $R^{5'}$ = | O, S, NH, $NC_1$-$C_2$-Alkyl, $CR^4_2$, CHOH, $CHOCH_3$, und $R^{5''}$ = $CR^4_2$, CHOH, $CHOCH_3$, wenn zwischen $R^{5'}$ und $R^{5''}$ eine Einfachbindung vorliegt, |
| $R^{5'}$ = $R^{5''}$ = | $CR^4$, wenn zwischen $R^{5'}$ und $R^{5''}$ eine Doppelbindung vorliegt, und |
| $R^6$, $R^{6'}$ = | jeweils unabhängig CH oder $CCH_3$. |

In einer bevorzugten Ausführungsform enthalten die erfindungsgemäßen Verbindungen Adenin oder Adenin-Analoga, wie z.B. $C_8$- und $N_6$-substituiertes Adenin, Deaza-Varianten wie 7-Deaza, Azavarianten wie 8-Aza oder Kombinationen wie 7-Deaza oder 8-Aza oder carbocyclische Analoga, wie Formycin, wobei die 7-Deazavarianten in der 7-

Position mit Halogen, $C_{1-6}$-Alkinyl, -Alkenyl oder -Alkyl substituiert sein können.

In einer weiteren bevorzugten Ausführungsform enthalten die Verbindungen Adenosin-Analoga, welche statt Ribose z.B. 2-Methoxydesoxyribose, 2'-Fluorodesoxyribose, Hexitol, Altritol bzw. polycyclische Analoga, wie Bicyclo-, LNA- und Tricyclo-Zucker enthalten.

Insbesondere können in den Verbindungen der Formel (II) auch (Di-)-Phosphatsauerstoffe isotronisch ersetzt sein, wie z.B. $O^-$ durch $S^-$ bzw. $BH_3^-$, O durch NH, $NCH_3$ bzw. $CH_2$ und =O durch =S. In den erfindungsgemäßen Verbindungen der Formel (II) ist W vorzugsweise $CONH_2$ oder $COCH_3$.

In den Gruppen der Formel (III) ist $R^5$ vorzugsweise $CH_2$. Weiterhin ist bevorzugt, dass $R^{5'}$ ausgewählt ist aus $CH_2$, CHOH und NH. In einer besonders bevorzugten Ausführungsform sind $R^{5'}$ und $R^{5''}$ jeweils CHOH. In noch einer weiteren bevorzugten Ausführungsform ist $R^{5'}$ NH und $R^{5''}$ $CH_2$. Am stärksten bevorzugt ist eine Verbindung der Formel (III), in welcher $R^4$ = H ist, $R^5 = CH_2$ ist, $R^{5'} = R^{5''}$ = CHOH ist, und $R^6 = R^{6'}$ = CH ist.

In der am stärksten bevorzugten Ausführungsform handelt es sich bei dem artifiziellen Coenzym um die literaturbekannte Verbindung carbaNAD (J.T. Slama, Biochemistry (1988), 27, 183 und Biochemistry (1989), 28, 7688). Andere stabile Coenzyme, welche erfindungsgemäß Anwendung finden können, sind in WO 98/33936, WO 01/49247, WO 2007/012494, US 5,801,006, US 11/460,366 und der Publikation Blackburn et al. (Chem. Comm. (1996), 2765), beschrieben, auf deren Offenbarung hiermit ausdrücklich Bezug genommen wird.

Das in dem erfindungsgemäßen Verfahren verwendete diagnostische Element kann ein beliebiges diagnostisches Element sein, welches eine die mutierte Dehydrogenase und das artifizielle Coenzym enthaltende trockene Reagenzienschicht umfasst und von der den Analyten enthaltenden Probe benetzt werden kann. Die Reagenzienschicht kann neben der mutierten Dehydrogenase und dem artifiziellen Coenzym ggf. weitere Reagenzien, welche dem qualitativen Nachweis oder der quantitativen Bestimmung des Analyten dienen, wie z.B. einen geeigneten Mediator, sowie geeignete Hilfs- oder/und Zusatzstoffe umfassen.

Vorzugsweise werden im Rahmen der vorliegenden Erfindung diagnostische Elemente eingesetzt, auf die der Analyt in Form einer wässrigen oder nichtwässrigen Lösung aufgebracht werden kann. In einer besonders bevorzugten Ausführungsform der Erfindung handelt es sich bei dem diagnostischen Element um ein Testband, eine Testdisc, ein Testpad, einen Teststreifen, eine Teststreifentrommel, oder um die in WO 2005/084530 A2 genannten diagnostischen Elemente, auf welche hiermit ausdrücklich Bezug genommen wird. Die in vorliegender Anmeldung beschriebenen diagnostischen Elemente umfassen hierbei jeweils mindestens einen Testbereich, welcher mit einer den Analyten enthaltenden Probe in Kontakt gebracht werden kann und unter Verwendung geeigneter Mittel eine qualitative oder/und quantitative Bestimmung des Analyten ermöglicht.

Der Begriff "Testband", wie er hierin verwendet wird, bezeichnet ein bandförmiges diagnostisches Element, welches üblicherweise mehr als einen einzelnen Testbereich, bevorzugt mindestens 10 einzelne Testbereiche, stärker bevorzugt mindestens 25 einzelne Testbereiche, und am stärksten bevorzugt mindestens 50 einzelne Testbereiche umfasst. Vorzugsweise sind die einzelnen Testbereiche jeweils in einem Abstand von wenigen Millimetern bis zu wenigen Zentimetern, beispielsweise in einem Abstand von < 2.5 cm, voneinander angeordnet, wobei das Testband zwischen aufeinander folgenden Testbereichen gegebenenfalls Markierungsbereiche zur Wegerfassung beim Bandtransport oder/und zur Kalibrierung umfassen kann. Derartige Testbänder sind beispielsweise in EP 1 739 432 A1 beschrieben, auf deren Offenbarung ausdrücklich Bezug genommen wird.

Der Begriff "Testdisc", wie er hierin verwendet wird, bezeichnet ein scheibenförmiges diagnostisches Element, welches einen oder mehrere einzelne Testbereiche, beispielsweise mindestens 10 einzelne Testbereiche, umfassen kann. In einer Ausführungsform ist die Testdisc mit einer dünnen Schicht der Testchemie, beispielsweise mit einer Schicht in einer Dicke von etwa 20 $\mu$m, überzogen, auf welche eine Probe des Analyten aufgebracht werden kann, wobei in Abhängigkeit vom Volumen der Probe ein mehr oder weniger großer Bereich der Testdisc von der Probe benetzt wird und zur Bestimmung des Analyten verwendet werden kann. Der nicht-benetzte Bereich der Testdisc, welcher infolge des Durchtritts von Feuchtigkeit durch die Testchemieschicht teilweise oder vollständig befeuchtet sein kann, steht in der Folge für weitere Bestimmungen des Analyten zur Verfügung.

Die erfindungsgemäße Verfahren kann zur Bestimmung einer beliebigen biologischen oder chemischen Substanz verwendet werden, welche photochemisch oder elektrochemisch nachweisbar ist. Vorzugsweise wird der Analyt aus der Gruppe bestehend aus Äpfelsäure, Alkohol, Ammonium, Ascorbinsäure, Cholesterin, Cystein, Glucose, Glucose-6-phosphat, Glutathion, Glycerin, Harnstoff, 3-Hydroxybutyrat, Milchsäure, 5'-Nukleotidase, Peptiden, Pyruvat, Salicylat und Triglyceriden ausgewählt, wobei Glucose besonders bevorzugt ist. Der Analyt kann dabei aus einer beliebigen Quelle stammen, ist jedoch bevorzugt in einem Körperfluid, umfassend, jedoch nicht beschränkt auf, Vollblut, Plasma, Serum, Lymphflüssigkeit, Gallenflüssigkeit, Cerebrospinalflüssigkeit, extrazelluläre Gewebeflüssigkeit, Harn, sowie Drüsensekrete wie z.B. Speichel oder Schweiß, enthalten. Bevorzugt wird mittels der hierin beschriebenen diagnostischen Elemente das Vorhandensein und/oder die Menge eines Analyten in einer Probe aus Vollblut, Plasma, Serum oder extrazellulärer Gewebeflüssigkeit bestimmt.

Die qualitative oder/und quantitative Bestimmung des Analyten kann auf beliebige Art und Weise erfolgen. Hierzu können grundsätzlich alle aus dem Stand der Technik bekannten Methoden zum Nachweis enzymatischer Reaktionen eingesetzt werden, welche ein messbares Signal erzeugen, das manuell oder unter Verwendung geeigneter Mittel ausgewertet bzw. ausgelesen werden kann. Im Rahmen der vorliegenden Erfindung gelangen bevorzugt optische Nachweismethoden, welche beispielsweise die Messung von Absorption, Fluoreszenz, Circulardichroismus (CD), optische Rotationsdispersion (ORD), Refraktometrie, etc. umfassen, sowie elektrochemische Techniken zur Anwendung. Besonders bevorzugt erfolgt die Bestimmung des Vorhandenseins oder/und der Menge des Analyten photometrisch oder fluorometrisch, beispielsweise indirekt über eine fluorometrisch detektierbare Veränderung des artifiziellen Coenzyms.

In einem weiteren Aspekt betrifft die Erfindung ein diagnostisches Element zur Bestimmung eines Analyten, umfassend eine trockene Reagenzienschicht, welche

(a) eine für den Analyten spezifische, mutierte Dehydrogenase, und
(b) ein artifizielles Coenzym enthält.

Was bevorzugte Ausgestaltungen des diagnostischen Elements sowie der darin enthaltenen mutierten Dehydrogenase bzw. des darin enthaltenen artifiziellen Coenzyms anbetrifft, so wird auf die Ausführungen in Zusammenhang mit der Beschreibung des erfindungsgemäßen Verfahrens verwiesen.

Die Erfindung soll durch die nachfolgenden Figuren und Beispiele näher erläutert werden:

Beschreibung der Figuren

Figur 1: Kinetik von Wildtyp-Glucose-Dehydrogenase aus Bacillus subtilis in Gegenwart von NAD/NADH als Coenzym bei Glucose-Konzentrationen von 0.0 mg/dl, 35.2 mg/dl, 54.2 mg/dl, 146.6 mg/dl, 249.0 mg/dl, 338.6 mg/dl und 553.6 mg/dl (von oben nach unten betrachtet).
**Figur 1A**: Enzymaktivität 1556.2 kU/100 g Masse
**Figur 1B:** Enzymaktivität 1004.0 kU/100 g Masse
**Figur 1C**: Enzymaktivität 502.0 kU/100 g Masse
**Figur 1D**: Enzymaktivität 251.0 kU/100 g Masse
**Figur 1E**: Enzymaktivität 25.10 kU/100 g Masse.

**Figur 2:** Kinetik einer durch Mutation von Wildtyp-Glucose-Dehydrogenase aus Bacillus subtilis erhaltenen Glucose-Dehydrogenase-Doppelmutante GlucDH_E170K_K252L in Gegenwart von carbaNAD/carbaNADH als Coenzym bei Glucose-Konzentrationen von 0.0 mg/dl, 34.4 mg/dl, 141.2 mg/dl, 236.6 mg/dl, 333.8 mg/dl und 525.8 mg/dl (von oben nach unten betrachtet). Enzymaktivität: 4.60 kU/100 g Masse.

**Figur 3:** Fluoreszenzspektrum des Komplexes Glucose-Dehydrogenase (GlucDH)/NADH vor und nach Titration mit Gluconolacton.

**Figur 4:** Fluoreszenzspektrum des Komplexes Glucose-Dehydrogenase (GlucDH)/NADH vor und nach Titration mit Glucose.

**Figur 5:** Kinetik der Umsetzung von Glucose in Gegenwart von Wildtyp-Glucose-Dehydrogenase und NADH bei verschiedenen Glucose-Konzentrationen.
**Figur 5A:** Kinetik ohne zusätzlich hinzugefügtes Gluconolacton bei Glucose-Konzentrationen von 77.0 mg/dl, 207.0 mg/dl, 300.0 mg/dl und 505.0 mg/dl (von oben nach unten betrachtet).
**Figur 5B:** Kinetik mit zusätzlich hinzugefügtem Gluconolacton bei Glucose-Konzentrationen von 96.2 mg/dl, 274.0 mg/dl, 399.0 mg/dl und 600.0 mg/dl (von oben nach unten betrachtet).

**Figur 6:** Darstellung der Aminosäuresequenz der Glucose-Dehydrogenase-Doppelmutante GlucDH_E170K_K252L.

**Beispiele**

Beispiel 1: Herstellung einer Doppelmutante von Glucose-Dehydrogenase aus Bacillus subtilis mit Aminosäuresubstitutionen E170K und K252L (GlucDH_E170K_K252L)

Zur Generierung eines gegenüber nativer Dehydrogenase stabilisierten Enzyms wurde die Nukleinsäuresequenz von Glucose-Dehydrogenase aus Bacillus subtilis im Plasmid pKK177 (kloniert über EcoRI und HindIII) eingesetzt.

Durch ortsspezifische Mutagenese zunächst an Position 170 und anschließende ortsspezifische Mutagenese an Position 252 der Aminosäuresequenz der Wildtyp-Glucose-Dehydrogenase wurden die Mutationen E170K und K252L eingeführt. Die jeweiligen Mutageneseschritte erfolgten mit Hilfe spezifisch designter Primer im Zuge einer PCR-Reaktion.

Das erhaltene PCR-Produkt wurde in Escherichia coli XL1blue MRF' transformiert. Die Zellen wurden ausplattiert, Plasmid enthaltende Klone wurden über Nacht angezogen, und die Enzymaktivität wurde vor bzw. nach Temperaturbelastung ermittelt (Stress-Test: 30 min bei 50°C). Die Ergebnisse sind in Tabelle 1 dargestellt.

Tabelle 1: Restaktivität von Wildtyp-Glucose-Dehydrogenase aus Bacillus subtilis und den Mutanten GlucDH_E170K bzw. GlucDH_E170K_K252L nach Stressbelastung (getestet in Escherichia coli XL1biue MRF')

| | Restaktivität nach Belastung (%) |
|---|---|
| Wildtyp-Glucose-Dehydrogenase | 23 |
| Mutante GlucDH_E170K | 80 |
| Mutante GlucDH_E170K_K252L | 130 |

Positive Klone wurden vor Sequenzierung zwei weitere Male getestet. Die auf diese Weise generierte Doppelmutante GlucDH_E170K_K252L wurde in den Produktionsstamm Escherichia coli NM522 mit pUBS-520 als Helferplasmid transformiert.

Beispiel 2: Aufreinigung der Doppelmutante GlucDH_E170K_K252L

Je 10 g Biomasse wurde in 50 ml eines 30 mM Kaliumphosphatpuffers pH 6.5 aufgenommen und bei ca. 800 bar aufgeschlossen. Nach Abtrennung der Zelltrümmer erfolgte eine Chromatographie an DEAE-Sepharose (Firma GE Healthcare) bei einer Beladung von < 40 mg Protein/ml Säulenvolumen und unter Verwendung eines linearen Gradienten von Puffer A (30 mM Kaliumphosphatpuffer pH 6.5) auf Puffer B (Puffer A + 500 mM NaCl). Die Fraktionen, welche Glucose-Dehydrogenase-Aktivität zeigten, wurden vereinigt und mit Ammoniumsulfat (Firma Aldrich) auf eine Leitfähigkeit von 230 mS/cm eingestellt.

Nach Zentrifugation wurde der blanke Überstand an Phenylsepharose FF (Firma GE Healthcare) bei einer maximalen Beladung von 10 mg Protein/ml Säulenvolumen chromatographisch getrennt. Die Elution erfolgte unter Verwendung eines linearen Gradienten von Puffer A, welcher mit Ammoniumsulfat auf eine Leitfähigkeit von 230 mS/cm eingestellt worden war, auf reinen Puffer A. Die Fraktionen wurden auf ihre Enzymaktivität hin getestet, vereinigt, auf eine Konzentration von ca. 50 mg/ml in 60 mM Kaliumphosphatpuffer pH 6.5 umgepuffert, konzentriert und lyophilisiert.

Beispiel 3: Bestimmung der Aktivität von Wildtyp-Glucose-Dehydrogenase aus Bacillus subtilis und der Doppelmutante GlucDH_E170K_K252L im Küvettentest

Um die spezifische Aktivität bzw. Lyophilisat-Aktivität von Wildtyp-Glucose-Dehydrogenase aus Bacillus subtilis sowie der in Beispiel 1 generierten Doppelmutante GlucDH_E170K_K252L in Gegenwart von NAD/NADH bzw. carbaNAD/carbaNADH zu untersuchen, wurde für beide Enzyme ein Glucose-Dehydrogenase-Aktivitätstest durchgeführt.

**Herstellung von Reagenzienlösungen**

Tris-Puffer (0.1 M, pH 8.5; 0.2 M NaCl)

11.68 g NaCl (Firma Sigma-Aldrich) und 12.11 g Tris (Firma Sigma-Aldrich) wurden in ca. 900 ml bidestilliertem Wasser gelöst, mit 1 N HCl auf einen pH-Wert von 8.5 eingestellt, und mit bidestilliertem Wasser auf 1000 ml aufgefüllt.

Verdünnungspuffer (3.8 mM NAD; 0.1 M Tris, pH 8.5; 0.2 M NaCl)

250 mg NAD (Firma Roche) wurden in 100 ml Tris-Puffer (0.1 M, pH 8.5; 0.2 M NaCl) gelöst.

Glucose-Lösung

2 g D(+)-Glucose-Monohydrat (Firma Sigma-Aldrich) wurden in 10 ml bidestilliertem Wasser gelöst. Nach 2 Stunden Standzeit bei Raumtemperatur und Einstellung des Mutarotationsgleichgewichts war die Lösung einsatzbereit.

NAD-Lösung (15 mM)

10 mg NAD (Firma Roche) wurden in 1 ml bidestilliertem Wasser gelöst..

carbaNAD-Lösung (15 mM)

10 mg carbaNAD (Firma Roche) wurden in 1 ml bidestilliertem Wasser gelöst.

**Probenvorbereitung**

Zur Vorbereitung der Messung wurden 10 mg des zu untersuchenden Enzyms in 1 ml Verdünnungspuffer gelöst und 60 min bei Raumtemperatur gehalten, um eine Rekonstitution zu gestatten. Anschließend wurde mit Verdünnungspuffer auf 0.12 bis 0.23 U/ml verdünnt.

**Durchführung der Messung**

Zur Durchführung der Messung wurden 1.35 ml Tris-Puffer, 0.1 ml Glucose-Lösung und 0.05 ml NAD-Lösung bzw. 0.05 ml carbaNAD-Lösung (jeweils auf 25°C temperiert) in eine Kunststoffküvette pipettiert, miteinander vermischt, und im Küvettenschlitten auf 25°C temperiert.

Nachdem sich die Extinktion der Lösung nicht mehr änderte (Leerwert), wurde die Reaktion durch Einbringen von 0.025 ml Probe in die Küvette in Gang gesetzt und die Extinktion der Probe über einen Zeitraum von 5 min verfolgt.

Meßwellenlänge: 340 nm
Testvolumen: 1.525 ml
Schichtdicke: 1 cm
Temperatur: 25°C
Auswertebereich: 1-5 min

**Auswertung**

Die Auswertung der Aktivität des jeweiligen Enzyms in wässriger Lösung erfolgte unter Verwendung der nachfolgenden Gleichung:

$$\text{Aktivität} = (1.525 \times \Delta A/\text{min} \times \text{Verdünnungsfaktor}) / (\varepsilon_{340} \times 0.025 \times 1)\ \text{U/ml}$$

wobei:

$\Delta A = A_t - A_0$ = Steigung der Absorptionsänderung über die Zeit
$\varepsilon_{340} = 6.3\ [l \times mmol^{-1} \times cm^{-1}]$

Die Ergebnisse der Bestimmung sind in Tabelle 2 wiedergegeben.

Tabelle 2: Aktivität von Wildtyp-Glucose-Dehydrogenase aus Bacillus subtilis (WT-GlucDH) und der Doppelmutante GlucDH_E170K_K252L

| | | WT-GlucDH | GlucDH_E170K_ K252L |
|---|---|---|---|
| NAD | U/mg Lyophilisat | 203 | 167 |
| | U/mg Enzym | 484 | 270 |
| | Km mM | 0.08 | 0.07 |
| | $V_{max}$ (U/mg Lyophilisat) | 122 | 144 |

(fortgesetzt)

| | | WT-GlucDH | GlucDH_E170K_ K252L |
|---|---|---|---|
| carbaNAD | U/mg Lyophilisat | 3.4 | 2.3 |
| | U/mg Enzym | 8.2 | 3.7 |
| | % U/mg Lyophilisat zu NAD | 1.7% | 1.4% |
| | Km mM | 0.3 | 1.4 |
| | $V_{max}$ (U/mg Lyophilisat) | 3 | 13 |

Wie aus Tabelle 2 hervorgeht, ist die Aktivität des Systems WT-GlucDH/carbaNAD (8.2 U/mg Enzym) unter standardisierten Bedingungen in einer Küvette um zwei Größenordnungen geringer als die Aktivität des Systems WT-GlucDH/NAD (484 U/mg Enzym). Gleichermaßen stellt man fest, dass die Aktivität der Doppelmutante GlucDH_E170K_K252L in Gegenwart des artifiziellen Coenzyms carbaNAD etwa zwei Größenordnungen niedriger liegt (3.7 U/mg Enzym) als in Gegenwart des nativen Coenzyms NAD (270 U/mg Enzym).

Beispiel 4: Bestimmung der Kinetik von Wildtyp-Glucose-Dehydrogenase aus Bacillus subtilis (WT-GlucDH) und der Doppelmutante GlucDH_E170K_K252L in einer trockenen Reagenzschicht

Zur Bestimmung der Kinetik von Enzymen wurden verschiedene Teststreifen hergestellt, welche entweder die native Glucose-Dehydrogenase aus Bacillus subtilis (WT-GlucDH) oder die in Beispiel 1 hergestellte Doppelmutante GlucDH_E170K_K252L in Kombination mit NAD/NADH bzw. carbaNAD/carbaNADH als Coenzym enthielten.

Im Einzelnen wurde zu diesem Zweck jeweils zunächst eine Teillösung 1, bestehend aus 18.4 g 1 M Phosphatpuffer pH 7.0, 1.4 g Gantrez S97 (Firma International Specialty Products), 2.94 g 16%ige NaOH-Lösung, 0.34 g Mega 8 (Firma Sigma-Aldrich), 0.039 g Geropon T77 (Firma Rhone-Poulenc) und 1.90 g Polyvinylpyrrolidon 25000 (Firma Fluka) hergestellt.

Diese Teillösung wurde anschließend mit einer Teillösung 2, bestehend aus 0.50 g Natriumchlorid, 21.3 g bidestilliertem Wasser, 4.43 g Transpafill (Firma Evonik) und 2.95 g Propiofan (Firma BASF), sowie einer über Nacht im Kühlschrank aufbewahrten Teillösung 3, bestehend aus 17.4 g 1 M Phosphatpuffer pH 7.0, 0.5 g Natriumchlorid, 14.35 g 2 M Dikaliumhydrogenphosphat und den in nachfolgender Tabelle 3 jeweils angegebenen Mengen an Dehydrogenase, Coenzym und ggf Rinderserumalbumin (BSA; Firma Roche), versetzt. Das enzymatisch inaktive Rinderserumalbumin wurde der Rezeptur im Falle des Einsatzes reduzierter Mengen an nativer Dehydrogenase hinzugefügt, um die Matrixeigenschaften des Teststreifens möglichst konstant zu halten.

Tabelle 3: Gehalt an Dehydrogenase bzw. Coenzym in den eingesetzten Teststreifen

| Enzym | Masse Enzym (g) | Lyophilisat-Aktivität (U/mg) | Coenzym | Masse Coenzym (g) | Masse BSA (g) | kU/100 g Masse |
|---|---|---|---|---|---|---|
| WT-GlucDH | 6.2 | 251 | NAD | 7.36 | 0 | 1556.2 |
| WT-GlucDH | 4.0 | 251 | NAD | 7.36 | 2.2 | 1004.0 |
| WT-GlucDH | 2.0 | 251 | NAD | 7.36 | 4.2 | 502.0 |
| WT-GlucDH | 1.0 | 251 | NAD | 7.36 | 5.2 | 251.0 |
| WT-GlucDH | 0.1 | 251 | NAD | 7.36 | 6.1 | 25.10 |
| GlucDH-Doppelmutante | 2.0 | 2.3 | carbaNAD | 2.1 | 0 | 4.60 |

Die auf diese Weise erhaltenen Teststreifen wurden auf Labormessgeräten (Eigenbau Firma Roche), welche eine Anregungs-LED (375 nm) und gängige Detektoren (BPW34 blueenhanced) umfassten, vermessen. Das aufgegebene Probenmaterial war Blut mit eingestellten Glucosewerten. Die Ergebnisse der Bestimmung sind in den Figuren 1 und 2 dargestellt.

Wie aus den Figuren 1A-1E ersichtlich ist, verschlechtert sich die Kinetik der Umsetzung von Glucose mittels Wildtyp-Glucose-Dehydrogenase in Gegenwart von NAD mit abnehmendem Enzymgehalt im Teststreifen und damit abnehmender Enzymaktivität. Insofern würde man erwarten, dass die Kinetik der Umsetzung von Glucose mittels der Doppelmutante GlucDH_E170K_K252L in Gegenwart von carbaNAD aufgrund der deutlich geringeren Enzymaktivität

von lediglich 4.60 kU/100 g Masse (siehe Tabelle 3) noch schlechtere Ergebnisse liefert.

Figur 2 zeigt die Kinetik der nach Beispiel 1 erhaltenen mutierten Glucose-Dehydrogenase in Gegenwart von carbaNAD als Coenzym. Wie aus Figur 2 hervorgeht, tritt die mit einer verringerten Enzymaktivität erwartete Verschlechterung der Kinetik nicht auf. Vielmehr zeigt die Doppelmutante in Gegenwart von carbaNAD eine bessere Kinetik als alle in Tabelle 3 aufgelisteten Rezepturen, welche die korrespondierende Wildtyp-Glucose-Dehydrogenase und das native Coenzym NAD enthalten.

Unter Berücksichtigung der in den Beispielen 3 und 4 beschriebenen Ergebnisse scheint für die Umsatzgeschwindigkeit von Glucose in trockenen Reagenzienschichten nicht die Aktivität des Enzyms, sondern vielmehr die Lage der Komplexbildung zwischen Enzym, Coenzym, reduziertem Coenzym, Glucose und Gluconolacton ausschlaggebend zu sein, welche im Falle der in Beispiel 1 hergestellten Mutante mit carbaNAD/carbaNADH offenkundig besser liegt als im Falle des Wildtyp-Enzyms mit nativem NAD/NADH.

<u>Beispiel 5:</u> Nachweis ternärer Komplexe bestehend aus Glucose-Dehydrogenase, NADH und Glucose bzw. Gluconolacton

Um die Existenz ternärer Komplexe bestehend aus Enzym, reduziertem Coenzym und Glucose bzw. Gluconolacton zu überprüfen, wurden im Küvettentest Versuche zur Bindung des Analyten basierend auf den Fluoreszenzeigenschaften von NADH durchgeführt.

Zu diesem Zweck wurden 1 mg NADH (Firma Roche) in 1 ml Phosphatpuffer gelöst und das zugehörige Fluoreszenzspektrum aufgenommen. Anschließend wurden 10 mg Wildtyp-Glucose-Dehydrogenase (GlucDH) aus Bacillus subtilis zugegeben, wobei sich der literaturbekannte Komplex GlucDH-NADH bildete, der infolge einer deutlich längeren Lebensdauer von NADH (3 ns im Gegensatz zu 0.4 ns im freien Zustand) ein verschobenes Emissionsmaximum bei 450 nm liefert (siehe Figur 3). Durch Titration dieses Komplexes mit Gluconolacton erniedrigte sich die Fluoreszenz bei gleichzeitiger Verschiebung des Emissionsmaximums auf 427 nm, was das Vorliegen einen neuen Komplexes anzeigt, bei welchem es sich um den ternären Komplex GlucDH-NADH-Gluconolacton handelt (siehe Figur 3).

Die Erniedrigung der Fluoreszenz, welche auch einer Erniedrigung der Lebensdauer entspricht, beruht vermutlich auf dem schnellen Energieabbau durch das Redoxpaar NADH-Gluconolacton. Titriert man nämlich den binären Komplex GlucDH-NADH mit Glucose, so bildet sich ein ineffektiver ternärer GlucDH-NADH-Glucose-Komplex, der mangels reduzierbarer Spezies keinen gesonderten Energieabbau betreiben kann und daher bei gleichem Emissionsmaximum eine eher noch höhere Lebensdauer und Intensität zeigt (siehe Figur 4).

Sofern für die Geschwindigkeit der Umsetzung von Glucose zu Gluconolacton in einer trockenen Reagenzienschicht die Spaltung des ternären Komplexes GlucDH-NADH-Gluconolacton und damit die Wiederverfügbarkeit des Enzyms ausschlaggebend ist, sollte eine Zugabe von Gluconolacton eine Verlangsamung der Umsetzung von Glucose bewirken, da zusätzliches Gluconolacton (zusätzlich zu dem bei der Umsetzung gebildeten Gluconolacton) weitere Enzymkomplexe inhibieren sollte.

Bei einer Kinetikmessung, bei der Blut in Abwesenheit (siehe Figur 5A) bzw. Gegenwart (siehe Figur 5B) von Gluconolacton auf eine trockene Reagenzienschicht gemäß Beispiel 4 der vorliegenden Anmeldung aufgegeben wurde, konnte diese Vermutung bestätigt werden. Figur 5B zeigt eine deutliche Verlangsamung der Umsetzung gegenüber der in Figur 5A vermessenen Probe.

## Patentansprüche

**1.** Verfahren zur Bestimmung von Glucose, umfassend die Schritte:

(a) Inkontaktbringen einer den Analyten enthaltenden Probe mit einem diagnostischen Element, umfassend eine trockene Reagenzienschicht, welche

(i) eine mutierte Glucose-Dehydrogenase (EC 1.1.1.47), und
(ii) ein artifizielles Coenzym enthält, und

(b) Bestimmen des Vorhandenseins oder/und der Menge des Analyten, wobei die mutierte Glucose-Dehydrogenase eine gegenüber dem korrespondierenden Wildtyp-Enzym (EC 1.1.1.47) erhöhte thermische oder hydrolytische Stabilität aufweist und eine Mutation an Position 170 oder/und Position 252 der Aminosäuresequenz der korrespondierenden Wildtyp-Glucose-Dehydrogenase umfasst, wobei die Mutation an Position 170 einen Aminosäureaustausch von Glutaminsäure gegen Arginin oder Lysin oder/und die Mutation an Position 252 einen Aminosäureaustausch von Lysin gegen Leucin umfasst und wobei das artifizielle Coenzym eine im Vergleich zum nativen Coenzym erhöhte hydrolytische Stabilität aufweist und als artifizielles Coenzym eine Verbindung der allgemeinen Formel (II) verwendet wird:

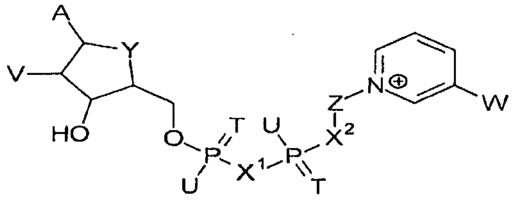

(II)

worin

A = Adenin oder ein Analogon davon,
T = jeweils unabhängig O, S,
U = jeweils unabhängig OH, SH, $BH_3^-$, $BCNH_2^-$,
V = jeweils unabhängig OH oder eine Phosphatgruppe, oder zwei Gruppen, die eine cyclische Phosphatgruppe bilden;
W = COOR, $CON(R)_2$, COR, $CSN(R)_2$ mit R = jeweils unabhängig H oder $C_{1-2}$-Alkyl,
$X^1$, $X^2$ = jeweils unabhängig O, $CH_2$, $CHCH_3$, $C(CH_3)_2$, NH, $NCH_3$,
Y = NH, S, O, $CH_2$,
Z = ein linearer oder cyclischer organischer Rest ist, mit der Maßgabe, dass Z und der Pyridin-Rest nicht durch eine glycosidische Verbindung verknüpft sind, oder ein Salz oder gegebenenfalls eine reduzierte Form davon.

2. Verfahren nach Anspruch 1,
**dadurch gekennzeichnet,**
**dass**, die Mutation an Position 170 einen Aminosäureaustausch von Glutaminsäure gegen Lysin umfasst.

3. Verfahren nach Anspruch 1 oder 2,
**dadurch gekennzeichnet,**
**dass** die mutierte Glucose-Dehydrogenase eine durch Mutation einer Wildtyp-Glucose-Dehydrogenase aus Bacillus megaterium, Bacillus subtilis oder Bacillus thuringiensis, insbesondere aus Bacillus subtilis, erhaltene Glucose-Dehydrogenase ist.

4. Verfahren nach einem der Ansprüche 1 bis 3,
**dadurch gekennzeichnet,**
**dass** die mutierte Glucose-Dehydrogenase die in SEQ ID NO: 1 dargestellte Aminosäuresequenz besitzt.

5. Verfahren nach einem der Ansprüche 1 bis 4
**dadurch gekennzeichnet,**
**dass** als artifizielles Coenzym carbaNAD verwendet wird.

6. Verfahren nach einem der Ansprüche 1 bis 5
**dadurch gekennzeichnet,**
**dass** als diagnostisches Element ein Teststreifen verwendet wird.

7. Verfahren nach einem der Ansprüche 1 bis 6,
**dadurch gekennzeichnet,**
**dass** die Bestimmung des Vorhandenseins oder/und der Menge des Analyten photometrisch oder fluorometrisch erfolgt.

8. Diagnostisches Element zur Bestimmung von Glucose, umfassend eine trockene Reagenzienschicht, welche

(a) eine mutierte Glucose-Dehydrogenase (EC 1.1.1.47), und
(b) ein artifizielles Coenzym enthält,
wobei die mutierte Glucose-Dehydrogenase eine gegenüber dem korrespondierenden Wildtyp-Enzym (EC

1.1.1.47) erhöhte thermische oder hydrolytische Stabilität aufweist und eine Mutation an Position 170 oder/und Position 252 der Aminosäuresequenz der korrespondierenden Wildtyp-Glucose-Dehydrogenase umfasst, wobei die Mutation an Position 170 einen Aminosäureaustausch von Glutaminsäure gegen Arginin oder Lysin oder/und die Mutation an Position 252 einen Aminosäureaustausch von Lysin gegen Leucin umfasst und wobei das artifizielle Coenzym eine im Vergleich zum nativen Coenzym erhöhte hydrolytische Stabilität aufweist und das artifizielle Coenzym eine Verbindung der allgemeinen Formel (II) ist:

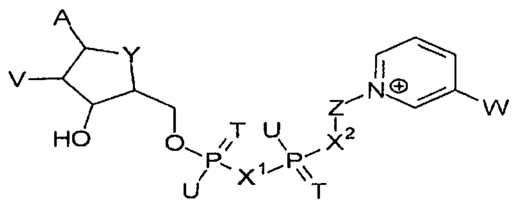

(II)

worin

A = Adenin oder ein Analogon davon,
T = jeweils unabhängig O, S,
U = jeweils unabhängig OH, SH, $BH_3^-$, $BCNH_2^-$,
V = jeweils unabhängig OH oder eine Phosphatgruppe, oder zwei Gruppen, die eine cyclische Phosphatgruppe bilden;
W = COOR, $CON(R)_2$, COR, $CSN(R)_2$ mit R = jeweils unabhängig H oder $C_{1-2}$-Alkyl,
$X^1$, $X^2$ = jeweils unabhängig O, $CH_2$, $CHCH_3$, $C(CH_3)_2$, NH, $NCH_3$,
Y = NH, S, O, $CH_2$,
Z = ein linearer oder cyclischer organischer Rest ist, mit der Maßgabe, dass Z und der Pyridin-Rest nicht durch eine glycosidische Verbindung verknüpft sind, oder ein Salz oder gegebenenfalls eine reduzierte Form davon.

**9.** Diagnostisches Element nach Anspruch 8,
**dadurch gekennzeichnet,**
**dass** die mutierte Dehydrogenase die in SEQ ID NO: 1 dargestellte Aminosäuresequenz besitzt.

## Claims

**1.** Method for determining glucose, comprising the steps:

(a) bringing a sample containing the analyte into contact with a diagnostic element comprising a dry reagent layer which contains

(i) a mutated glucose dehydrogenase (EC 1.1.1.47), and
(ii) an artificial coenzyme, and

(b) determining the presence or/and the quantity of the analyte, wherein the mutated glucose dehydrogenase has an increased thermal or hydrolytic stability compared with the corresponding wild-type enzyme (EC 1.1.1.47) and comprises a mutation at position 170 or/and position 252 of the amino acid sequence of the corresponding wild-type glucose dehydrogenase, wherein the mutation at position 170 comprises an amino acid substitution of glutamic acid for arginine or lysine or/and the mutation at position 252 comprises an amino acid substitution of lysine for leucine and wherein the artificial coenzyme has an increased hydrolytic stability compared with the native coenzyme and a compound having the general formula (II) is used as the artificial coenzyme:

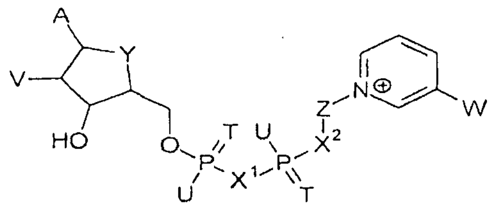

(II)

where

A = adenine or an analogue thereof,
T = in each case independently, O, S,
U = in each case independently, OH, SH, $BH_3^-$, $BCNH_2^-$,
V = in each case independently, OH or a phosphate group, or two groups which form a cyclic phosphate group;
W = COOR, $CON(R)_2$, COR, $CSN(R)_2$ and R = in each case independently, H or $C_{1-2}$-alkyl,
$X^1$, $X^2$ = in each case independently, O, $CH_2$, $CHCH_3$, $C(CH_3)_2$, NH, $NCH_3$,
Y = NH, S, O, $CH_2$
Z = a linear or cyclic organic group, with the condition that Z and the pyridine group are not linked by a glycosidic bond, or a salt or, if applicable, a reduced form thereof.

2. Method according to claim 1, **characterised in that** the mutation at position 170 comprises an amino acid substitution of glutamic acid for lysine.

3. Method according to claim 1 or 2, **characterised in that** the mutated glucose dehydrogenase is a glucose dehydrogenase obtained by mutation of a wild-type glucose dehydrogenase from Bacillus megaterium, Bacillus subtilis or Bacillus thuringiensis, in particular from Bacillus subtilis.

4. Method according to one of the claims 1 to 3, **characterised in that** the mutated glucose dehydrogenase has the amino acid sequence shown in SEQ ID NO: 1.

5. Method according to one of the claims 1 to 4, **characterised in that** carbaNAD is used as the artificial coenzyme.

6. Method according to one of the claims 1 to 5, **characterised in that** a test strip is used as the diagnostic element.

7. Method according to one of the claims 1 to 6, **characterised in that** the determination of the presence or/and the quantity of the analyte takes place photometrically or fluorometrically.

8. Diagnostic element for determining glucose, comprising a dry reagent layer which contains

(a) a mutated glucose dehydrogenase (EC 1.1.1.47), and
(b) an artificial coenzyme,
wherein the mutated glucose dehydrogenase has an increased thermal or hydrolytic stability compared with the corresponding wild-type enzyme (EC 1.1.1.47) and comprises a mutation at position 170 or/and position 252 of the amino acid sequence of the corresponding wild-type glucose dehydrogenase, wherein the mutation at position 170 comprises an amino acid substitution of glutamic acid for arginine or lysine or/and the mutation at position 252 comprises an amino acid substitution of lysine for leucine and wherein the artificial coenzyme has an increased hydrolytic stability compared with the native coenzyme and the artificial coenzyme is a compound having the general formula (II):

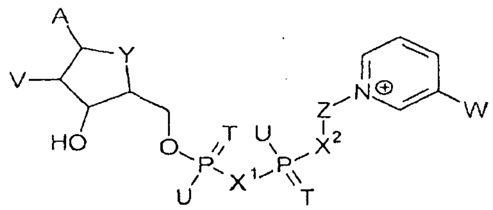

(II)

where

A = adenine or an analogue thereof,
T = in each case independently, O, S,
U = in each case independently, OH, SH, $BH_3^-$, $BCNH_2^-$,
V = in each case independently, OH or a phosphate group, or two groups which form a cyclic phosphate group;
W = COOR, $CON(R)_2$, COR, $CSN(R)_2$ and R = in each case independently, H or $C_{1-2}$-alkyl,
$X^1$, $X^2$ = in each case independently, O, $CH_2$, $CHCH_3$, $C(CH_3)_2$, NH, $NCH_3$,
Y = NH, S, O, $CH_2$
Z = a linear or cyclic organic group, with the condition that Z and the pyridine group are not linked by a glycosidic bond, or a salt or, if applicable, a reduced form thereof.

9. Diagnostic element according to claim 8, **characterised in that** the mutated dehydrogenase has the amino acid sequence shown in SEQ ID NO: 1.

## Revendications

1. Procédé de dosage de glucose, comprenant les étapes consistant à :

(a) mettre en contact un échantillon contenant l'analyte avec un élément diagnostique comprenant une couche sèche de réactifs laquelle contient

(i) une glucose-déshydrogénase (EC 1.1.1.47) mutée, et
(ii) une coenzyme artificielle, et

(b) déterminer la présence ou/et la quantité de l'analyte, la glucose-déshydrogénase mutée présentant une stabilité thermique et hydrolytique qui est supérieure par rapport à l'enzyme correspondante (EC 1.1.1.47) de type sauvage et comprenant une mutation à la position 170 ou/et la position 252 dans la séquence d'acides aminés de la glucose-déshydrogénase correspondante de type sauvage, ladite mutation à la position 170 comprenant un échange d'acide aminé consistant à remplacer l'acide glutamique par l'arginine ou la lysine ou/et la mutation à la position 252 comprenant un échange d'acide aminé consistant à remplacer la lysine par la leucine, et la coenzyme artificielle présentant une stabilité hydrolytique qui est supérieure par rapport à la coenzyme native, la coenzyme artificielle mise en oeuvre étant un composé répondant à la formule générale (II):

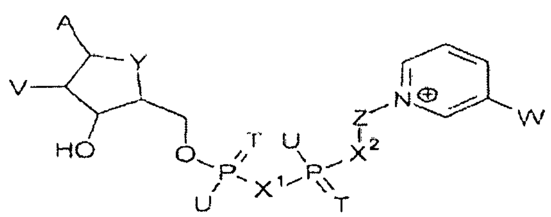

(II)

dans laquelle

A = adénine ou un analogue de celle-ci,
T = O, S, de manière indépendante,
U = OH, SH, $BH_3^-$, $BCNH_2^-$, de manière indépendante,
V = OH, ou un groupe phosphate, ou deux groupes formant un groupe phosphate cyclique, de manière indépendante,
W = COOR, $CON(R)_2$, COR, $CSN(R)_2$,
avec R = H ou $C_{1-2}$-alkyle, de manière indépendante,
$X^1$, $X^2$ = O, $CH_2$, $CHCH_3$, $C(CH_3)_2$, NH, $NCH_3$, de manière indépendante,
Y = NH, S, O, $CH_2$,
Z = un radical organique linéaire ou cyclique, à condition que Z et le radical de pyridine ne soient pas liés par une liaison glycosidique, ou bien un sel de celui-ci ou, éventuellement, une forme ayant subi une réduction.

**2.** Procédé selon la revendication 1,
**caractérisé en ce que**
la mutation à la position 170 comprend un échange d'acide aminé consistant à remplacer l'acide glutamique par la lysine.

**3.** Procédé selon les revendications 1 ou 2,
**caractérisé en ce que**
la glucose-déshydrogénase mutée est une glucose-déshydrogénase obtenue par mutation d'une glucose-déshydrogénase de type sauvage laquelle est issue de Bacillus megaterium, Bacillus subtilis ou Bacillus thuringiensis, notamment de Bacillus subtilis.

**4.** Procédé selon l'une des revendications 1 à 3,
**caractérisé en ce que**
la glucose-déshydrogénase mutée possède la séquence d'acides aminés représentée dans la SEQ-ID-Nr. 1.

**5.** Procédé selon l'une des revendications 1 à 4,
**caractérisé en ce que**
l'on met en oeuvre, en tant que coenzyme artificielle, la carbaNAD.

**6.** Procédé selon l'une des revendications 1 à 5,
**caractérisé en ce que**
l'on met en oeuvre, en tant qu'élément diagnostique, une bandelette d'essai.

**7.** Procédé selon l'une des revendications 1 à 6,
**caractérisé en ce que**
la présence ou/et la quantité de l'analyte est/sont déterminée(s) par photométrie ou fluorométrie.

**8.** Elément diagnostique destiné au dosage de glucose, comprenant une couche sèche de réactifs laquelle contient

(a) une glucose-déshydrogénase (EC 1.1.1.47) mutée, et
(b) une coenzyme artificielle,
la glucose-déshydrogénase mutée présentant une stabilité thermique et hydrolytique qui est supérieure par rapport à l'enzyme correspondante (EC 1.1.1.47) de type sauvage et comprenant une mutation à la position 170 ou/et la position 252 dans la séquence d'acides aminés de la glucose-déshydrogénase correspondante de type sauvage, ladite mutation à la position 170 comprenant un échange d'acide aminé consistant à remplacer l'acide glutamique par l'arginine ou la lysine ou/et la mutation à la position 252 comprenant un échange d'acide aminé consistant à remplacer la lysine par la leucine, et la coenzyme artificielle présentant une stabilité hydrolytique qui est supérieure par rapport à la coenzyme native, la coenzyme artificielle étant un composé répondant à la formule générale (II):

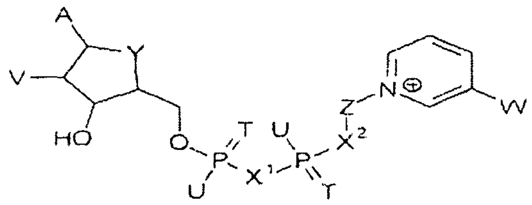

(II)

dans laquelle

A = adénine ou un analogue de celle-ci,
T = O, S, de manière indépendante,
U = OH, SH, $BH_3^-$, $BCNH_2^-$, de manière indépendante,
V = OH, ou un groupe phosphate, ou deux groupes formant un groupe phosphate cyclique, de manière indépendante,
W = COOR, $CON(R)_2$, COR, $CSN(R)_2$,
avec R = H ou $C_{1-2}$-alkyle, de manière indépendante,
$X^1$, $X^2$ = O, $CH_2$, $CHCH_3$, $C(CH_3)_2$, NH, $NCH_3$, de manière indépendante,
Y = NH, S, O, $CH_2$,
Z = un radical organique linéaire ou cyclique, à condition que Z et le radical de pyridine ne soient pas liés par une liaison glycosidique, ou un sel de celui-ci ou, éventuellement, une forme ayant subi une réduction.

**9.** Elément diagnostique selon la revendication 8,
**caractérisé en ce que**
la glucose-déshydrogénase mutée possède la séquence d'acides aminés représentée dans la SEQ-ID-Nr. 1.

Figur 1A

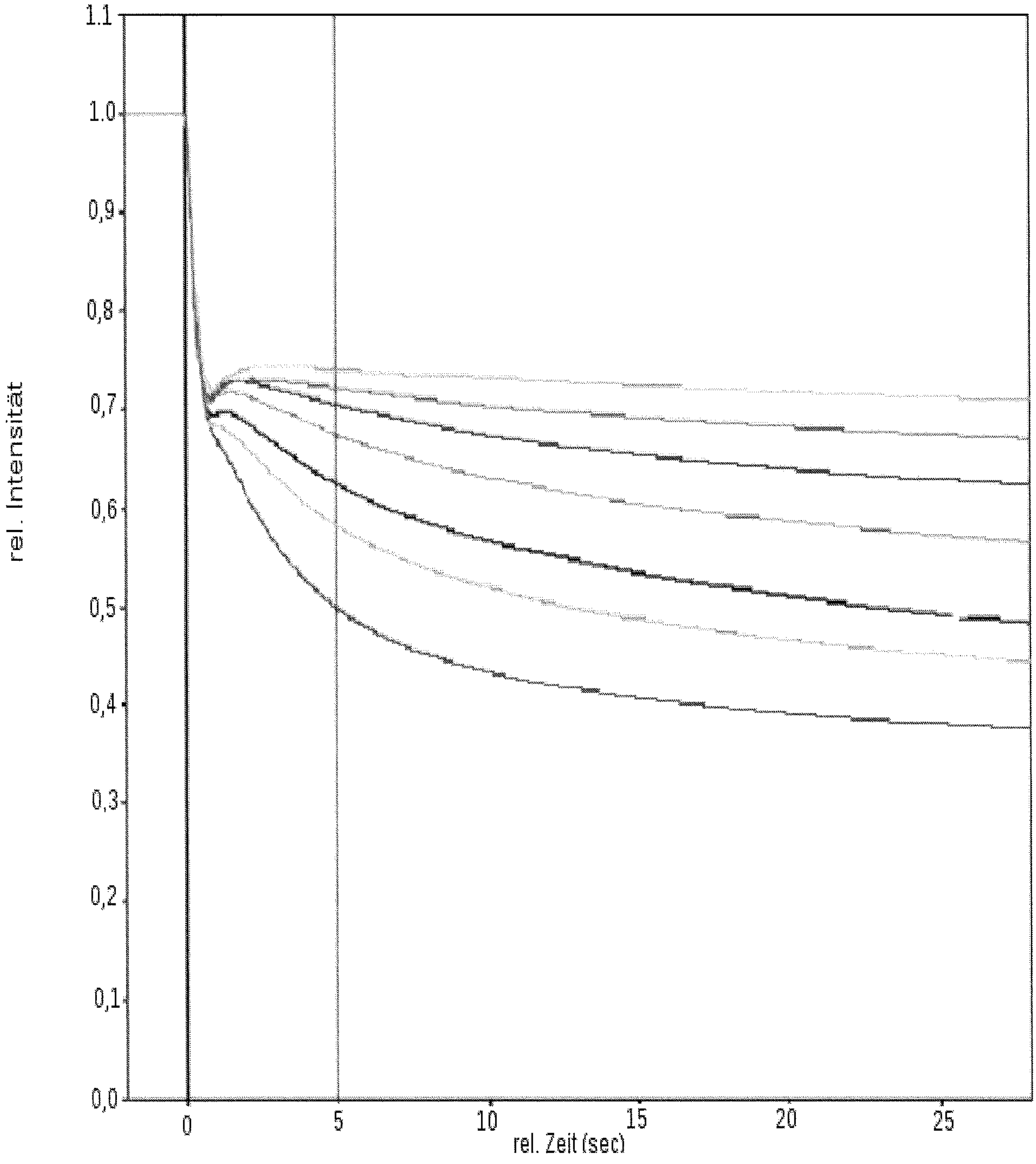
1.1
1.0
0,9
0,8
0,7
0,6
0,5
0,4
0,3
0,2
0,1
0,0
rel. Intensität
0
5
10
15
20
25
rel. Zeit (sec)

**Figur 1B**

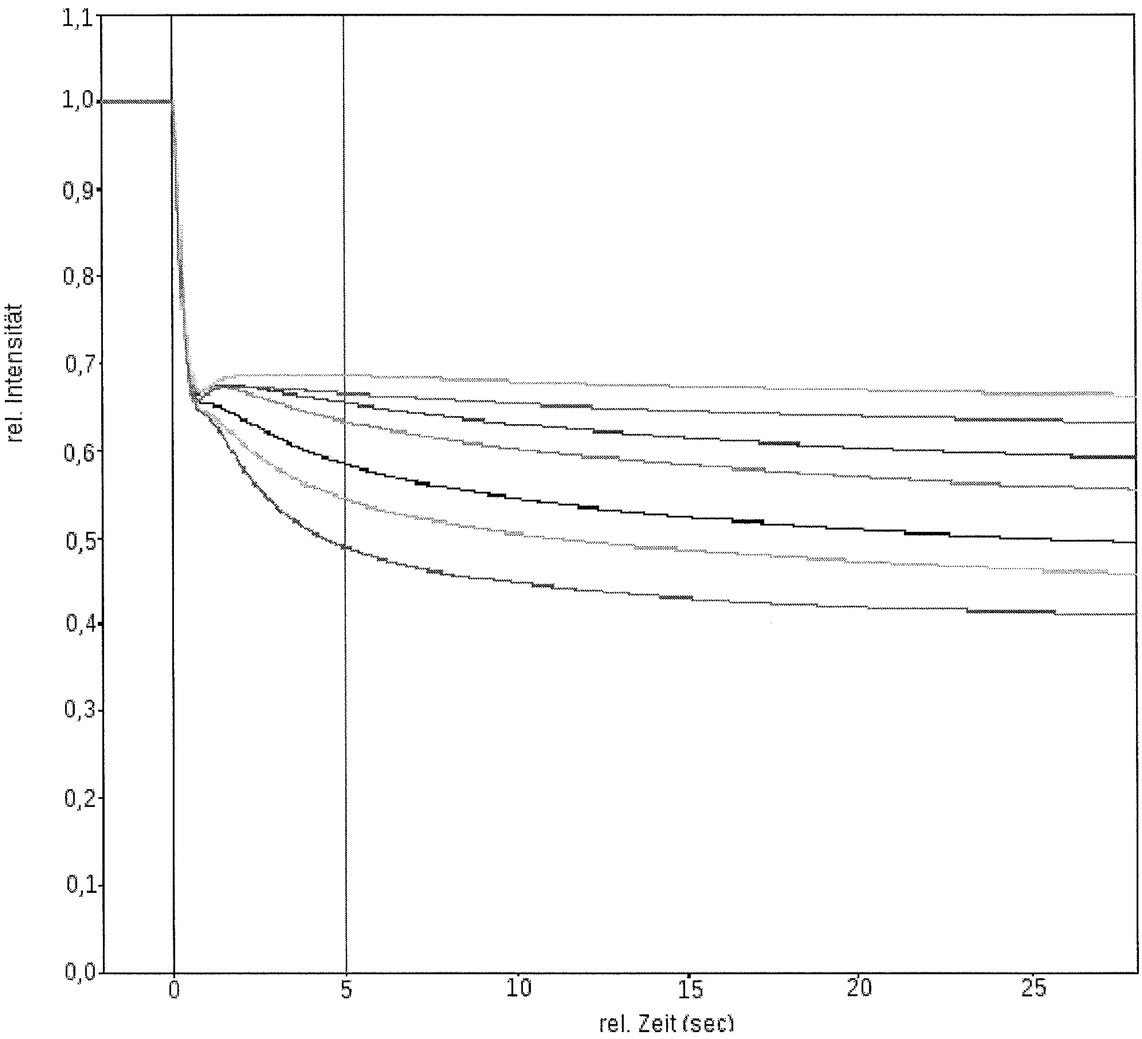
1,1
1,0
0,9
0,8
0,7
0,6
0,5
0,4
0,3
0,2
0,1
0,0
rel. Intensität
0
5
10
15
20
25
rel. Zeit (sec)

Figur 1C

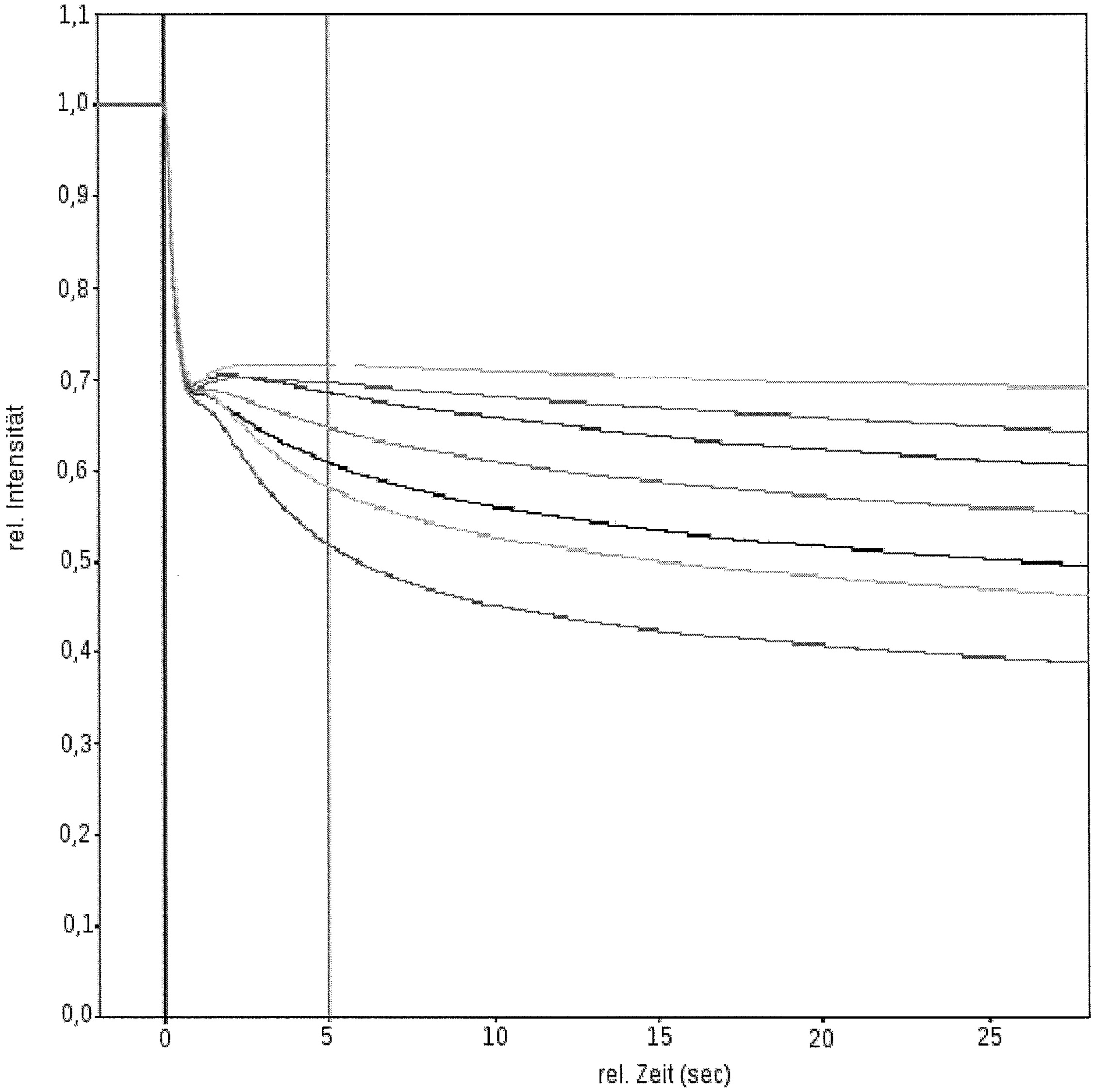
1,1
1,0
0,9
0,8
0,7
0,6
0,5
0,4
0,3
0,2
0,1
0,0
rel. Intensität
0
5
10
15
20
25
rel. Zeit (sec)

Figur 1D

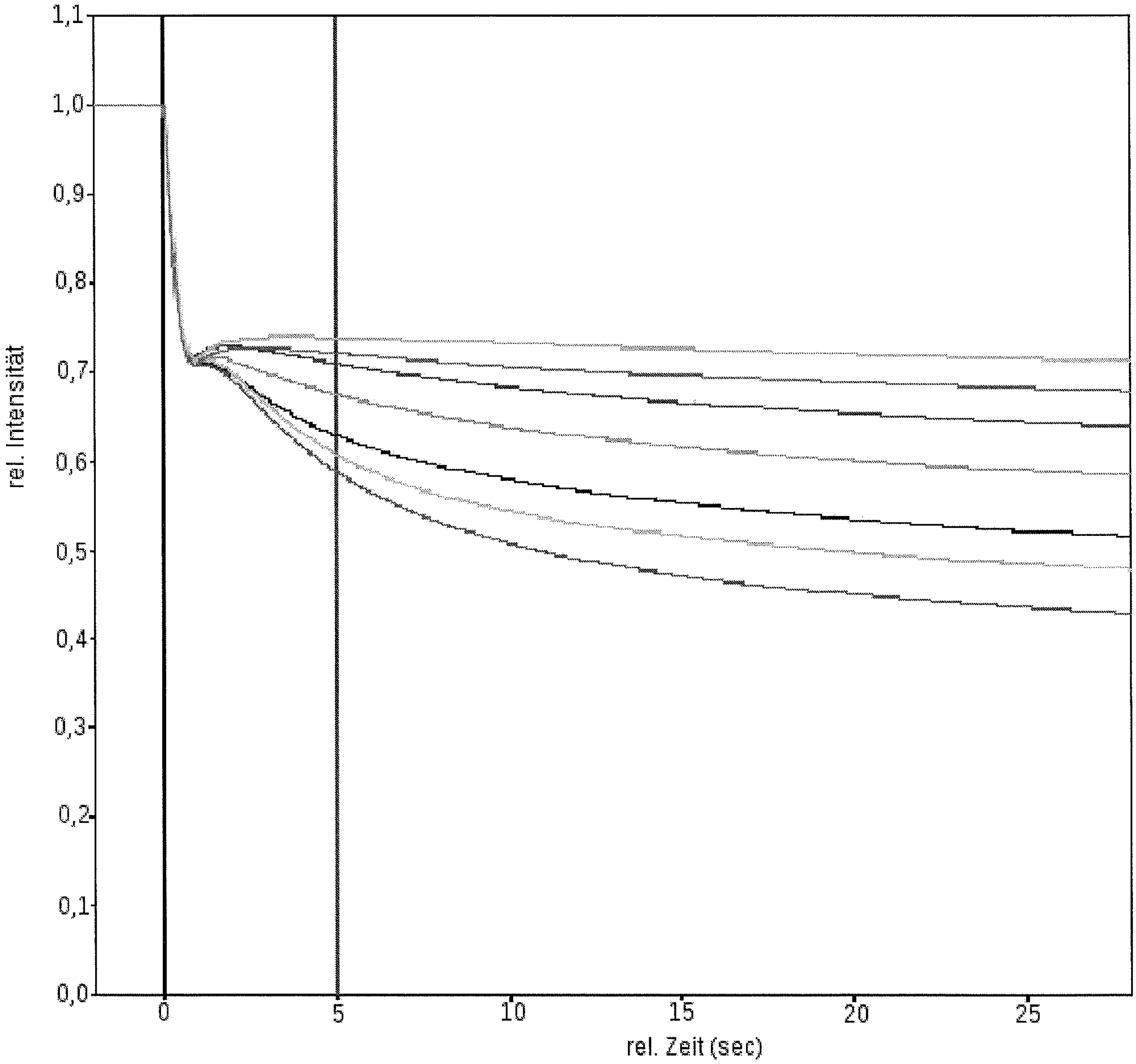
1,1
1,0
0,9
0,8
0,7
0,6
0,5
0,4
0,3
0,2
0,1
0,0
rel. Intensität
0
5
10
15
20
25
rel. Zeit (sec)

Figur 1E

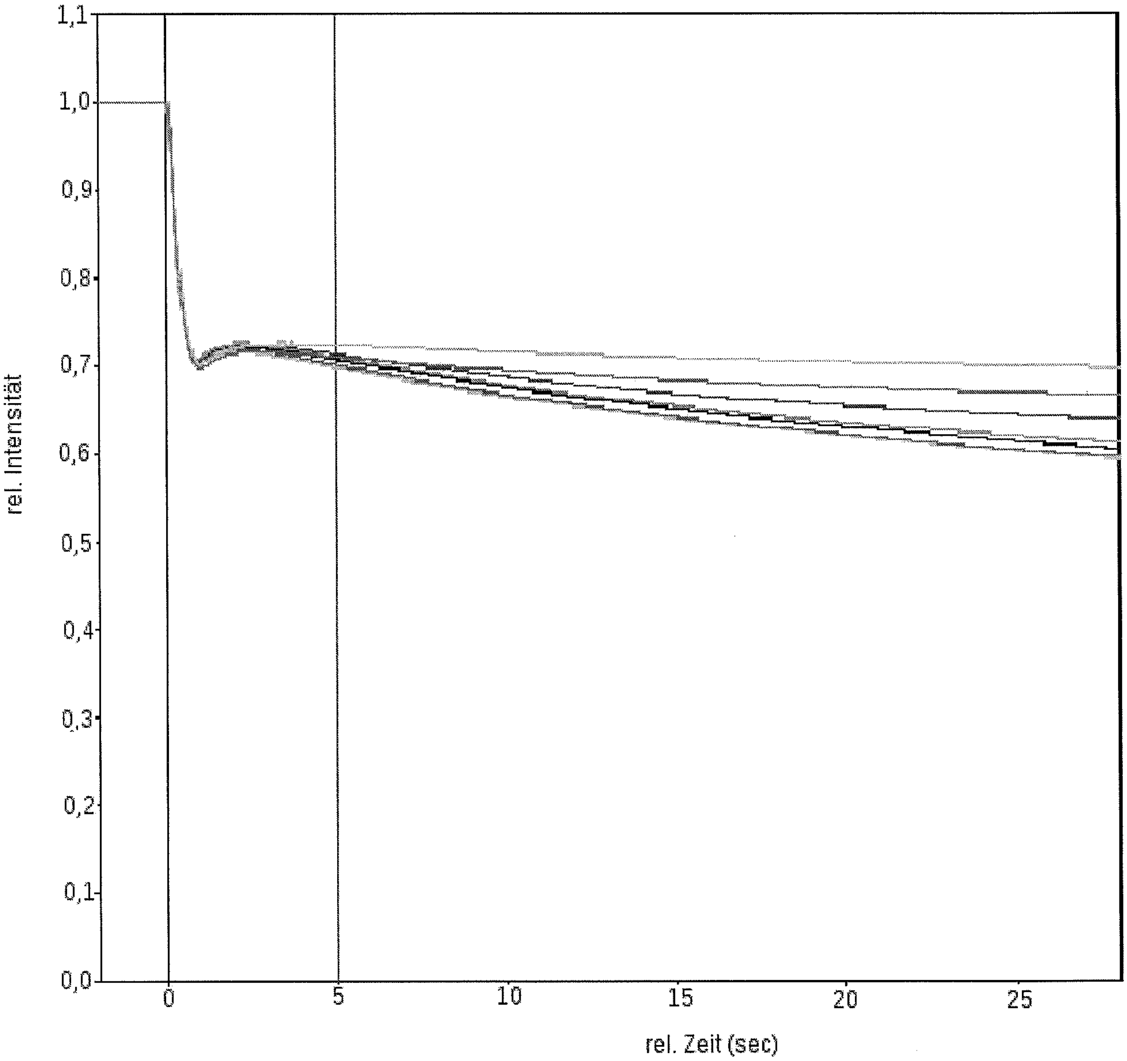
1,1
1,0
0,9
0,8
0,7
0,6
0,5
0,4
0,3
0,2
0,1
0,0
rel. Intensität
0
5
10
15
20
25
rel. Zeit (sec)

**Figur 2**

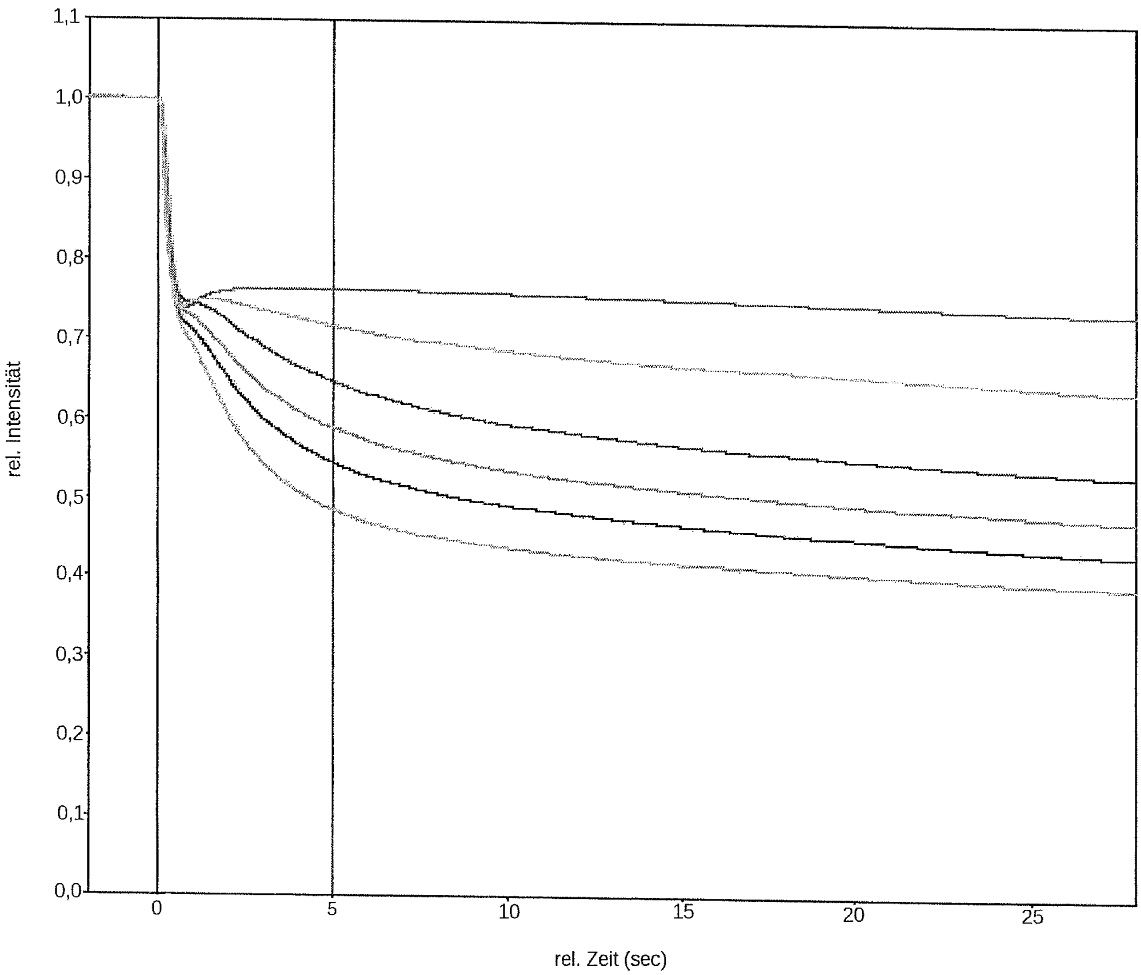
1,1
1,0
0,9
0,8
0,7
0,6
0,5
0,4
0,3
0,2
0,1
0,0
rel. Intensität
0
5
10
15
20
25
rel. Zeit (sec)

Figur 3

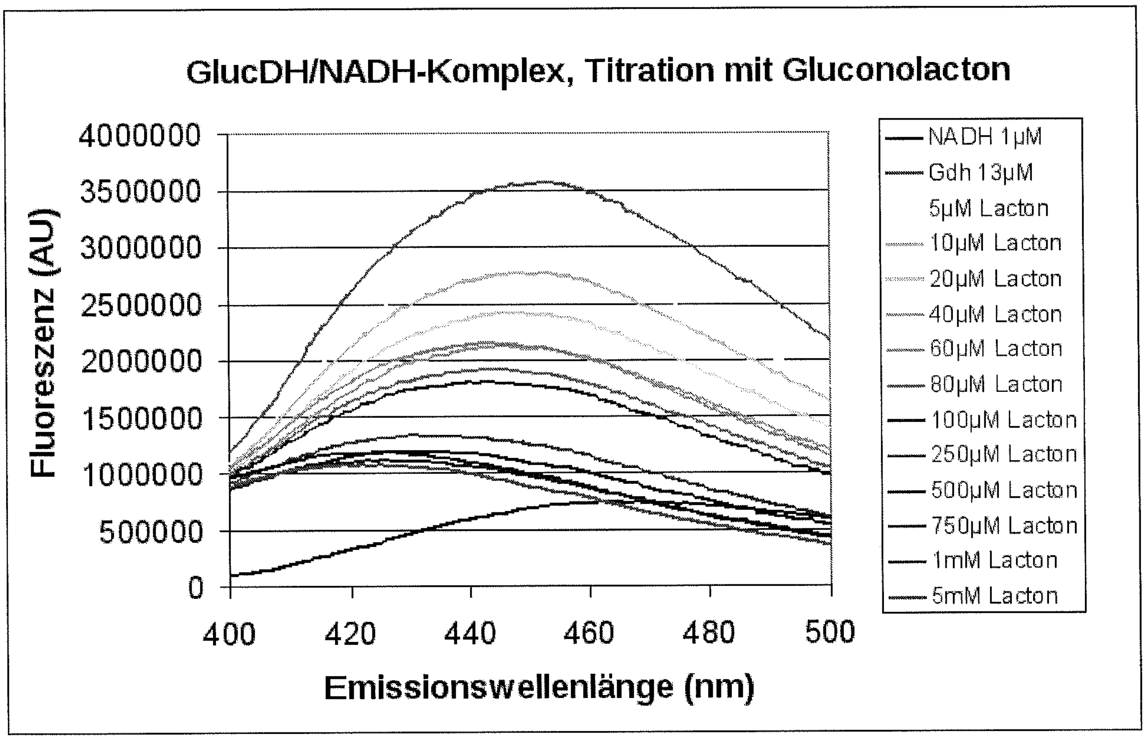
GlucDH/NADH-Komplex, Titration mit Gluconolacton
4000000
3500000
3000000
2500000
2000000
1500000
1000000
500000
0
Fluoreszenz (AU)
400
420
440
460
480
500
Emissionswellenlänge (nm)
NADH 1µM
Gdh 13µM
5µM Lacton
10µM Lacton
20µM Lacton
40µM Lacton
60µM Lacton
80µM Lacton
100µM Lacton
250µM Lacton
500µM Lacton
750µM Lacton
1mM Lacton
5mM Lacton

Figur 4

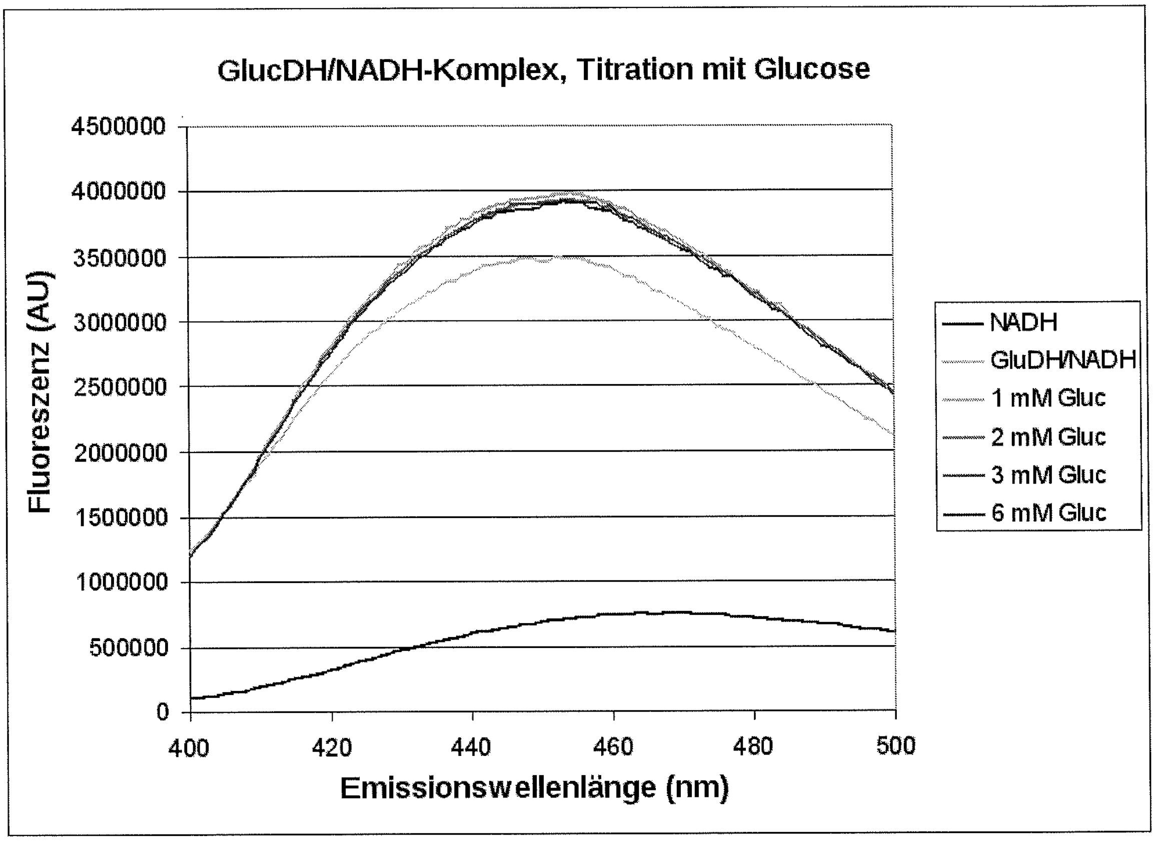
GlucDH/NADH-Komplex, Titration mit Glucose
4500000
4000000
3500000
3000000
2500000
2000000
1500000
1000000
500000
0
Fluoreszenz (AU)
400
420
440
460
480
500
Emissionswellenlänge (nm)
NADH
GluDH/NADH
1 mM Gluc
2 mM Gluc
3 mM Gluc
6 mM Gluc

**Figur 5A**

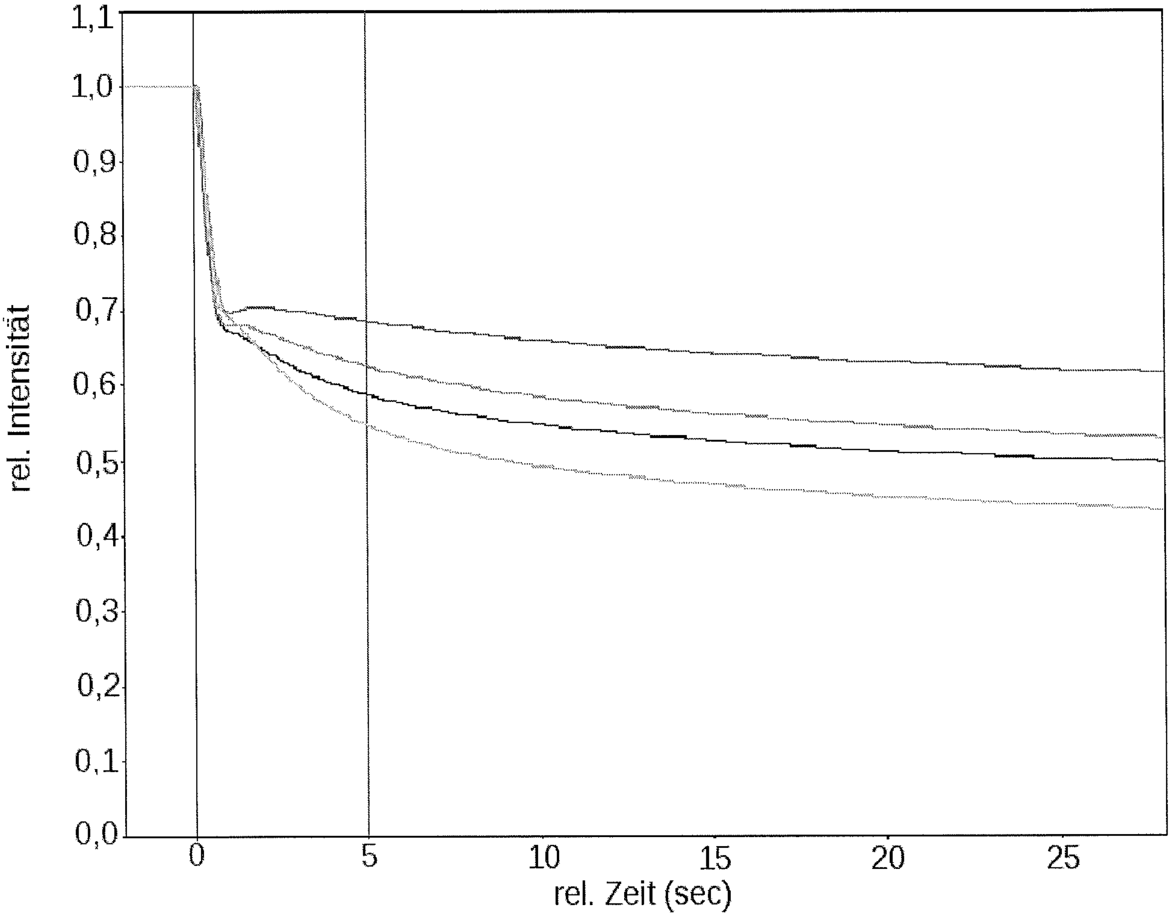
1,1
1,0
0,9
0,8
0,7
0,6
0,5
0,4
0,3
0,2
0,1
0,0
rel. Intensität
0
5
10
15
20
25
rel. Zeit (sec)

Figur 5B

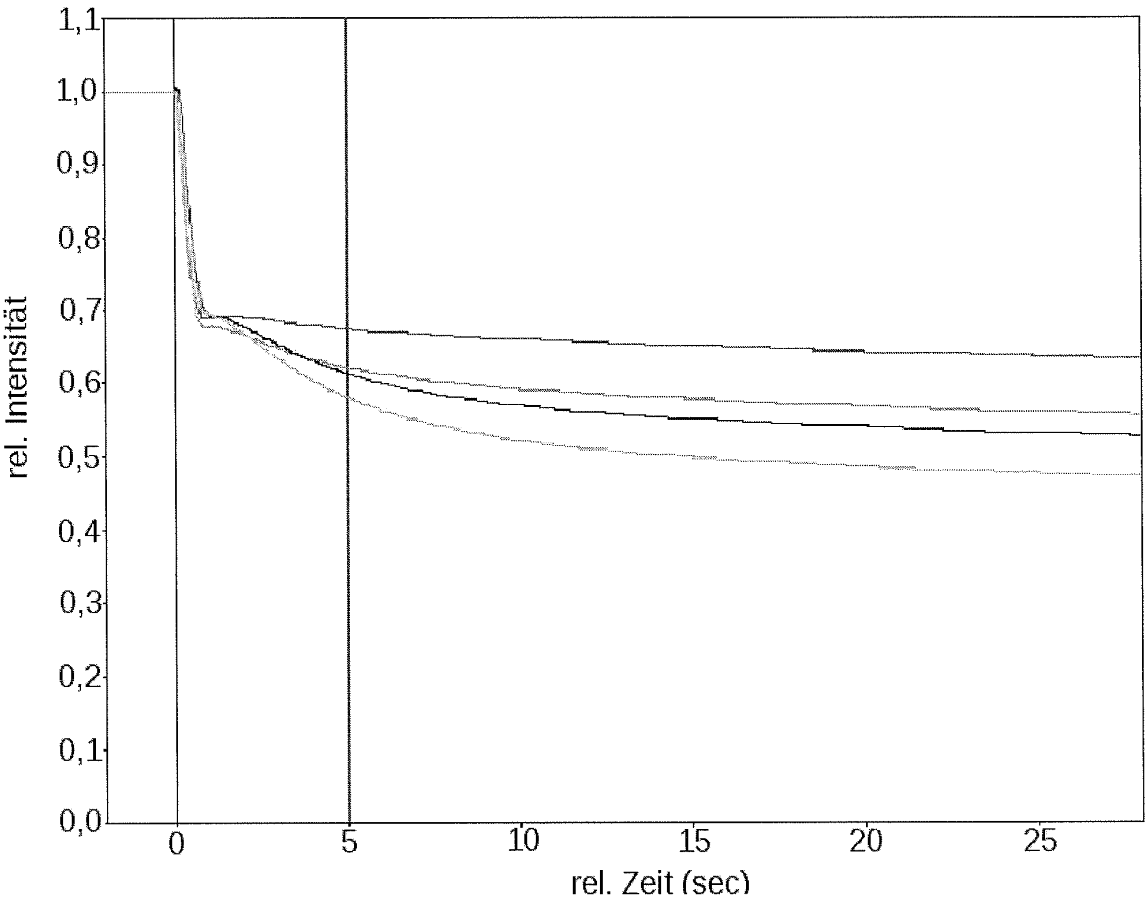
1,1
1,0
0,9
0,8
0,7
0,6
0,5
0,4
0,3
0,2
0,1
0,0
rel. Intensität
0
5
10
15
20
25
rel. Zeit (sec)

**Figur 6**

GlucDH_E170K_K252L

M Y P D L K G K V V A I T G A A S G L G K A M A I R F G K E
Q A K V V I N Y Y S N K Q D P N E V K E E V I K A G G E A V
V V Q G D V T K E E D V K N I V Q T A I K E F G T L D I M I
N N A G L E N P V P S H E M P L K D W D K V I G T N L T G A
F L G S R E A I K Y F V E N D I K G N V I N M S S V H E V I
P W P L F V H Y A A S K G G I K L M T K T L A L E Y A P K G
I R V N N I G P G A I N T P I N A E K F A D P K Q K A D V E
S M I P M G Y I G E P E E I A V A V W L A S K E S S Y V T
G I T L F A D G G M T L Y P S F Q A G R G

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2005045016 A2
- WO 9833936 A
- WO 0149247 A
- WO 2007012494 A
- US 5801006 A
- US 11460366 B
- WO 2005084530 A2
- EP 1739432 A1
- EP 2009001206 W


### In der Beschreibung aufgeführte Nicht-Patentliteratur


- **BAIK et al.** *Appl. Environ. Microbiol.,* 2005, vol. 71, 3285
- **VÁZQUEZ-FIGUEROA et al.** *ChemBioChem,* 2007, vol. 8, 2295
- **BAIK.** *Appl. Environ. Microbiol.,* 2005, vol. 71, 3285
- **VÁSQUEZ-FIGUEROA.** *ChemBioChem,* 2007, vol. 8, 2295
- **J.T. SLAMA.** *Biochemistry,* 1988, vol. 27, 183
- *Biochemistry,* 1989, vol. 28, 7688
- **BLACKBURN et al.** *Chem. Comm.,* 1996, 2765

SEQUENCE LISTING

<110> F. Hoffmann - La Roche AG Roche Diagnostics GmbH

<120> Schnelle Reaktionskinetik von Enzymen mit niedriger Aktivität in Trockenschichten

<130> 44103P WO

<150> PCT/EP2009/001206
<151> 2009-02-19

<160> 1

<170> PatentIn version 3.3

<210> 1

<211> 261
<212> PRT
<213> Artificial

<220>
<223> Mutant of glucose dehydrogenase from Bacillus subtilis

<220>
<221> MUTAGEN
<222> (170)..(170)

<220>
<221> MUTAGEN
<222> (252)..(252)

<400> 1

```
Met Tyr Pro Asp Leu Lys Gly Lys Val Val Ala Ile Thr Gly Ala Ala
1               5                   10                  15

Ser Gly Leu Gly Lys Ala Met Ala Ile Arg Phe Gly Lys Glu Gln Ala
            20                  25                  30

Lys Val Val Ile Asn Tyr Tyr Ser Asn Lys Gln Asp Pro Asn Glu Val
        35                  40                  45

Lys Glu Glu Val Ile Lys Ala Gly Gly Glu Ala Val Val Val Gln Gly
    50                  55                  60

Asp Val Thr Lys Glu Glu Asp Val Lys Asn Ile Val Gln Thr Ala Ile
65                  70                  75                  80

Lys Glu Phe Gly Thr Leu Asp Ile Met Ile Asn Asn Ala Gly Leu Glu
                85                  90                  95

Asn Pro Val Pro Ser His Glu Met Pro Leu Lys Asp Trp Asp Lys Val
            100                 105                 110

Ile Gly Thr Asn Leu Thr Gly Ala Phe Leu Gly Ser Arg Glu Ala Ile
```

```
            115                 120                 125

Lys Tyr Phe Val Glu Asn Asp Ile Lys Gly Asn Val Ile Asn Met Ser
    130                 135                 140

Ser Val His Glu Val Ile Pro Trp Pro Leu Phe Val His Tyr Ala Ala
145                 150                 155                 160

Ser Lys Gly Gly Ile Lys Leu Met Thr Lys Thr Leu Ala Leu Glu Tyr
                165                 170                 175

Ala Pro Lys Gly Ile Arg Val Asn Asn Ile Gly Pro Gly Ala Ile Asn
            180                 185                 190

Thr Pro Ile Asn Ala Glu Lys Phe Ala Asp Pro Lys Gln Lys Ala Asp
        195                 200                 205

Val Glu Ser Met Ile Pro Met Gly Tyr Ile Gly Glu Pro Glu Glu Ile
    210                 215                 220

Ala Ala Val Ala Val Trp Leu Ala Ser Lys Glu Ser Ser Tyr Val Thr
225                 230                 235                 240

Gly Ile Thr Leu Phe Ala Asp Gly Gly Met Thr Leu Tyr Pro Ser Phe
                245                 250                 255

Gln Ala Gly Arg Gly
            260
```